# FASCICULE DE BREVET EUROPEEN

(11) **EP 4 633 564 B1**
(45) Date de publication et mention de la délivrance du brevet: **25.11.2026**
(21) Numéro de dépôt: 23821307.8
(22) Date de dépôt: 12.12.2023
(51) Int. Cl.: A61F 9/008

(54) **SYSTEME DE DECOUPE D'UN TISSU OCULAIRE EN PORTIONS ELEMENTAIRES**
SYSTEM ZUM SCHNEIDEN VON AUGENGEWEBE IN ELEMENTARE TEILE
SYSTEM FOR CUTTING OCULAR TISSUE INTO ELEMENTARY PORTIONS

(30) Priorité: 12.12.2022 FR 2213179
(43) Date de publication de la demande: 22.10.2025
(73) Titulaire: KERANOVA, 42000 Saint Etienne (FR)
(72) Inventeur: BAUBEAU, Emmanuel, 42000 SAINT ETIENNE (FR); BERNARD, Aurélien, 42000 SAINT ETIENNE (FR)
(74) Mandataire: Be IP
(86) Numéro de dépôt international: PCT/EP2023/085289
(87) Numéro de publication internationale: WO 2024/126456

(56) Documents cités:
- WO-A1-2016/055539
- WO-A1-2018/020144
- WO-A1-2022/090408
- WO-A1-2022/207900
- US-A1- 2010 292 678
- US-A1- 2014 350 531
- US-A1- 2015 164 689
- US-A1- 2017 128 259
- US-A1- 2019 314 194

## Description

### DOMAINE TECHNIQUE

La présente invention concerne le domaine technique des opérations chirurgicales réalisées au laser femtoseconde, et plus particulièrement celui de la chirurgie ophtalmologique, notamment pour des applications de découpes de cornées, ou de cristallins.

L'invention concerne un dispositif de découpe d'un tissu humain ou animal, telle qu'une cornée, ou un cristallin, au moyen d'une source laser femtoseconde.

Par source laser femtoseconde, on entend une source lumineuse, apte à émettre un faisceau laser sous forme d'impulsions ultra-courtes, dont la durée est comprise entre 1 femtoseconde et 100 picosecondes, de préférence comprise entre 1 et 1000 femtosecondes, notamment de l'ordre de la centaine de femtosecondes.

### ART ANTERIEUR

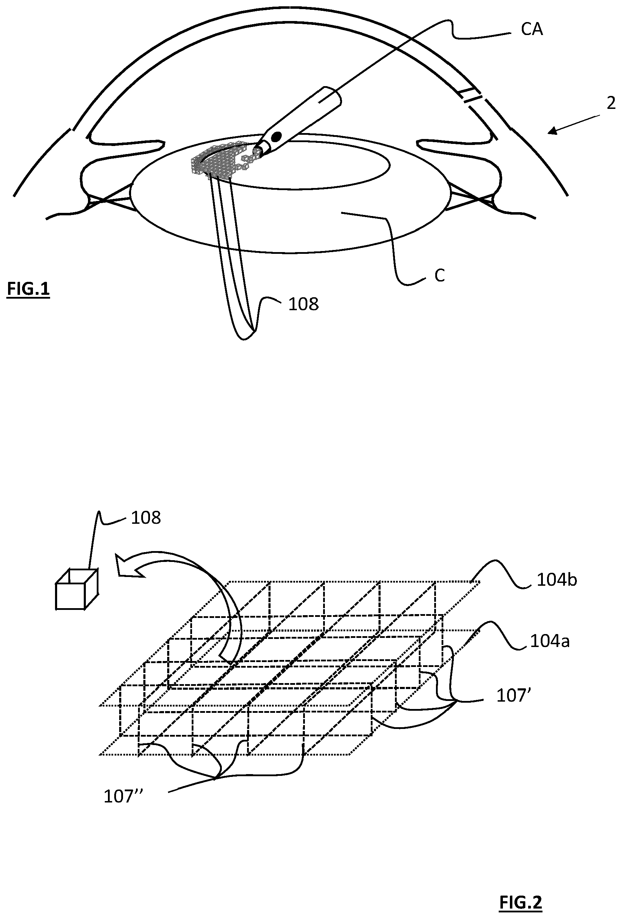

Dans le cadre de certaines chirurgies, telles qu'une chirurgie de la cataracte, il est souhaitable de subdiviser un tissu, tel que le cristallin C - en petites particules telles que des cubes 108 pour faciliter son extraction, par exemple à l'aire d'une canule d'aspiration CA, comme illustré à la figure 1.

Pour partitionner le cristallin en cubes 108 pouvant être aspirés par une canule d'aspiration, des plans de découpe horizontaux 104a, 104b et verticaux 107 peuvent être formés, comme illustré à la figure 2. Ces plans sont formés en commençant par le plan de découpe horizontal 104a le plus profond dans le cristallin et en empilant les plans de découpe verticaux 107 et horizontaux 104b successifs jusqu'au plan de découpe horizontal le plus superficiel dans le cristallin.

Le document WO 2022/090408 décrit notamment un appareil de découpe permettant la réalisation de plans de découpe horizontaux et verticaux. Cet appareil de découpe comprend :
- une source laser femtoseconde pour émettre un faisceau laser gaussien sous forme d'impulsions,
- un système de mise en forme positionné en aval de la source laser pour moduler la phase du front d'onde du faisceau laser gaussien, le système de mise en forme comprenant un modulateur spatial de lumière (SLM) et étant configuré pour produire un faisceau laser modulé à partir du faisceau laser gaussien,
- un scanner optique de balayage disposé en aval du système de mise en forme pour déplacer le faisceau laser modulé,
- un système optique de focalisation en aval du scanner optique de balayage, pour focaliser le faisceau laser modulé dans un plan focal de l'appareil de découpe et pour déplacer le plan focal en une pluralité de positions le long d'un axe optique de propagation du faisceau laser modulé,
- une unité de commande configurée pour piloter la source laser femtoseconde, le système de mise en forme, le scanner optique de balayage, et le système optique de focalisation, afin de réaliser des plans de découpe horizontaux et verticaux successifs.

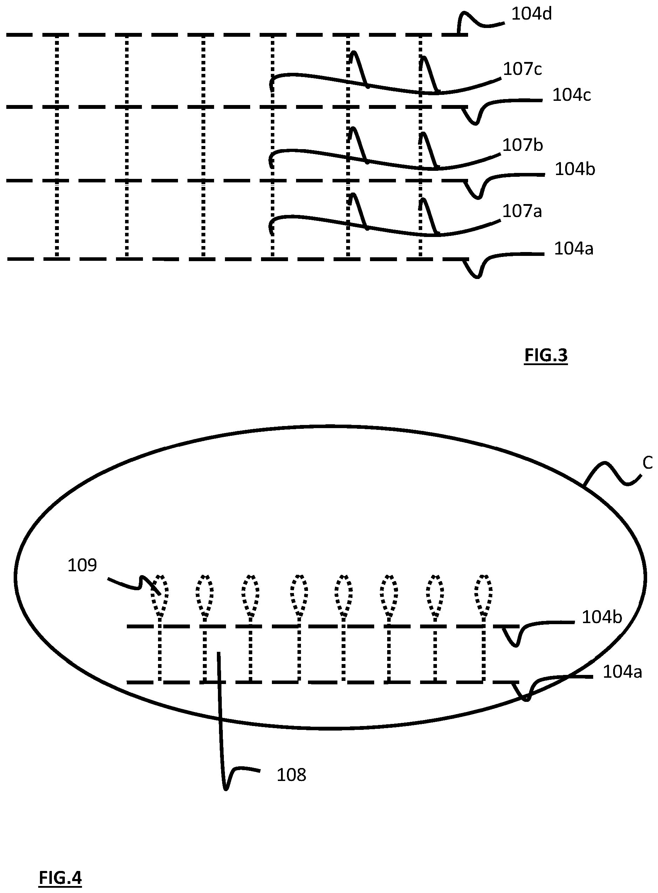

En référence à la figure 3, le principe de fonctionnement d'un tel appareil de découpe est le suivant.

Dans une première étape, un plan de découpe horizontal initial 104a (i.e. le plus profond) est réalisé. L'unité de commande :
- applique un masque de phase multipoints au système de mise en forme pour produire un faisceau laser modulé multipoints permettant de générer simultanément une pluralité de points d'impact,
- commande le déplacement du système de focalisation pour faire coïncider le plan focal de l'appareil de découpe avec le plan de découpe initial souhaité,
- active la source laser femtoseconde, et
- pilote le déplacement du scanner optique de balayage le long du chemin optique (par exemple en créneau).

Une succession de tirs est réalisée dans le plan focal de l'appareil de découpe. A chaque tir, plusieurs points d'impact - formant un motif - focalisent simultanément dans le plan focal. Chaque point d'impact forme une bulle de gaz. Le scanner optique permet de déplacer le faisceau laser modulé multipoints - et donc le motif - dans le plan focal entre chaque tir. Lorsque toute la surface du plan de découpe horizontal est recouverte de bulles de gaz, le plan de découpe horizontal initial 104a est finalisé.

Dans une deuxième étape, plusieurs plans de découpe verticaux adjacents 107a sont réalisés. Pour chaque plan de découpe vertical, l'unité de commande :
- applique un masque de phase conique (i.e. permettant d'appliquer une modulation de phase linéaire avec symétrie de rotation) au système de mise en forme pour produire un faisceau laser modulé de Bessel,
- commande le déplacement du système de focalisation pour focaliser le faisceau laser modulé à une profondeur désirée,
- active la source laser femtoseconde, et
- pilote le déplacement du scanner optique de balayage le long du chemin optique (par exemple un segment).

Une succession de tirs est réalisée. A chaque tir, une ligne d'impact se forme. Chaque ligne d'impact génère une bulle de gaz oblongue selon l'axe optique de propagation du faisceau laser modulé. Le scanner optique permet de déplacer le faisceau laser modulé - et donc la ligne d'impact - entre chaque tir. Si la profondeur de la ligne d'impact est inférieure à la profondeur souhaitée pour le plan de découpe vertical, alors l'unité de commande 60 peut contrôler le système optique de focalisation 50 pour faire varier la profondeur du plan focal de l'appareil de découpe.

Les plans de découpe verticaux 107a sont terminés lorsque tout le chemin de déplacement est recouvert de bulles de gaz oblongues.

Les étapes de réalisation de plans horizontaux 104b, 104c, 104d et verticaux 107b, 107c sont réitérées pour réaliser un empilement de cubes de cristallin 108.

Lorsque la multitude de bulles de gaz a été formée dans les différents plans horizontaux et verticaux, les cubes de cristallin ainsi formés peuvent être désolidarisés des portions de tissu non découpées en décrochant les ponts tissulaires existants entre les bulles de gaz à l'aide d'un outil.

Toutefois, la formation d'empilement de cubes 108 induit l'accumulation de gaz dans la partie supérieure de l'empilement de plans, comme illustré à la figure 4. Plus précisément, au fur et à mesure de la formation de cubes 108 empilés, les bulles de gaz 109 formées dans la profondeur du tissu 2 se déplacent vers la région la plus superficielle du tissu.

Cette accumulation de gaz peut remonter au-dessus du plan de découpe laser dans le tissu - par exemple dans la chambre antérieure de l'œil dans le cas d'une découpe de cristallin. Ceci peut induire des problèmes de pénétration du faisceau laser dans le tissu. En effet, les bulles de gaz forment une barrière de bulles opaque empêchant la propagation de l'énergie issue du faisceau laser sous celles-ci, et donc la découpe du tissu dans les régions situées sous cette accumulation de gaz.

Cette accumulation de gaz peut également provoquer une déformation du tissu durant la découpe qui peut induire un défaut de dimensionnement des cubes rendant l'aspiration de ceux-ci à partir d'une canule difficile.

Le document WO 2022/207900 décrit un appareil de découpe d'un tissu par génération de bulles de gaz oblongues, l'appareil incluant une source laser configurée pour émettre un faisceau laser gaussien et un dispositif de traitement du faisceau laser gaussien, le dispositif de traitement incluant un système de mise en forme sur lequel une consigne de modulation parabolique est appliquée afin de produire un faisceau laser modulé de type « Ligne Focale » à partir du faisceau laser gaussien.

Le document US 2010/292,678 décrit un système pour émettre des motifs de tir laser vers un cristallin.

Le document US 2014/350531 décrit un procédé de chirurgie laser photodisruptif comprenant les étapes de sélection d'une région cible d'un tissu, d'orientation d'un faisceau d'impulsions laser vers la région cible sélectionnée, et de formation de cellules dans la région cible sélectionnée.

Un but de la présente invention est de proposer un dispositif de découpe d'un tissu humain ou animal permettant d'éviter la formation d'une accumulation de gaz susceptible d'empêcher la propagation de l'énergie du faisceau laser sous ladite accumulation.

Plus précisément, un but de la présente invention est de proposer un dispositif de découpe permettant de réaliser une découpe complète d'un tissu - notamment d'un tissu oculaire tel qu'une cornée ou un cristallin - en évitant le masquage du faisceau laser au cours de la découpe.

Un autre but de la présente invention est de proposer un dispositif de découpe d'un tissu humain ou animal permettant de former des cubes de tissu de tailles plus homogènes.

### EXPOSE DE L'INVENTION

A cet effet l'invention propose un appareil de découpe d'un tissu humain ou animal, ledit appareil incluant une source laser femtoseconde configurée pour émettre un faisceau laser gaussien sous forme d'impulsions et un dispositif de traitement du faisceau laser gaussien, le dispositif de traitement étant disposé en aval de la source laser femtoseconde, le dispositif de traitement comprenant :
- un système de mise en forme positionné sur la trajectoire du faisceau laser gaussien, pour moduler la phase du front d'onde du faisceau laser gaussien, le système de mise en forme comprenant un modulateur spatial de lumière et étant configuré pour produire un faisceau laser modulé à partir du faisceau laser gaussien,
- un scanner optique de balayage disposé en aval du système de mise en forme pour déplacer le faisceau laser modulé,
- un système optique de focalisation en aval du système de mise en forme, pour focaliser le faisceau laser modulé dans un plan focal de l'appareil de découpe et pour déplacer le plan focal de l'appareil de découpe en une pluralité de positions le long d'un axe optique de propagation du faisceau laser modulé,
***remarquable en ce que*** le dispositif de traitement comprend en outre une unité de commande pour piloter la source laser femtoseconde, le système de mise en forme, le scanner optique de balayage, et le système optique de focalisation afin de réaliser des plans de découpe horizontaux et verticaux successifs, les plans de découpe horizontaux s'étendant perpendiculairement à l'axe optique et les plans de découpe verticaux s'étendant parallèlement à l'axe optique, ladite unité de commande étant configurée pour :
- commander la réalisation d'un premier plan de découpe horizontal,
- commander la réalisation d'une première pluralité de plans de découpe verticaux au-dessus du premier plan de découpe horizontal,
- commander la réalisation d'un deuxième plan de découpe horizontal au-dessus de la première pluralité de plans de découpe verticaux, le premier plan de découpe horizontal étant plus profond dans le tissu que le deuxième plan de découpe horizontal,
- commander la réalisation d'une deuxième pluralité de plans verticaux au-dessus du deuxième plan horizontal, dans laquelle la deuxième pluralité de plans de découpe verticaux est décalée latéralement (selon au moins une direction perpendiculaire à l'axe optique) relativement à la première pluralité de plans verticaux.

On entend, dans le cadre de la présente invention, par *« plan de découpe horizontal »,* un plan situé dans le tissu à traiter et s'étendant perpendiculairement à l'axe optique de propagation du faisceau laser issu de l'appareil de découpe.

On entend, dans le cadre de la présente invention, par « *plan de découpe vertical »,* un plan situé dans le tissu à traiter et s'étendant parallèlement à un axe optique de propagation du faisceau laser issu de l'appareil de découpe.

On entend, dans le cadre de la présente invention, par *« point d'impact »* une zone ponctuelle du faisceau laser comprise dans le plan focal de l'appareil de découpe et dans laquelle l'intensité du faisceau laser est suffisante pour générer une bulle de gaz dans un tissu.

On entend, dans le cadre de la présente invention, par *« ligne d'impact »* une zone linéaire du faisceau laser s'étendant perpendiculairement au plan focal de l'appareil de découpe (i.e. un segment du faisceau laser s'étendant parallèlement à l'axe optique) et dans laquelle l'intensité dudit faisceau laser est suffisante pour générer une bulle de gaz oblongue dans le tissu.

On entend, dans le cadre de la présente invention, par *« points d'impact adjacents »,* deux points d'impact disposés en regard l'un de l'autre et non séparés par un autre point d'impact. On entend par *« points d'impact voisins* » deux points d'un groupe de points adjacents entre lesquels la distance est minimale.

On entend, dans le cadre de la présente invention, par *« motif »* une pluralité de points d'impact laser générés simultanément.

Ainsi, l'invention permet de générer plusieurs groupes de plans de découpe verticaux séparés par des plans de découpe horizontaux pour former des cubes élémentaires de tissus pouvant être aspirés par une canule d'aspiration. Avantageusement, chaque groupe de plans de découpe verticaux est décalé latéralement relativement aux groupes de plans de découpe verticaux qui lui sont adjacents.

En particulier, chaque groupe est défini par une pluralité de plans de découpe verticaux formés entre deux plans de découpe horizontaux. Dans l'hypothèse où trois plans de découpe horizontaux superposés sont ménagés dans le tissu, à savoir :
- un plan de découpe horizontal profond,
- un plan de découpe horizontal intermédiaire, et
- un plan de découpe horizontal superficiel,
alors deux groupes de plans de découpe verticaux sont formés entre les plans de découpe horizontaux :
- un premier groupe de plans de découpe verticaux est formé entre le plan de découpe horizontal profond et le plan de découpe horizontal intermédiaire, et
- un deuxième groupe de plans verticaux est formé entre le plan de découpe intermédiaire et le plan de découpe superficiel, les plans de découpe verticaux du deuxième groupe étant décalés latéralement relativement aux plans de découpe verticaux du premier groupe.

Ce décalage latéral entre plans de découpe verticaux de groupe adjacent permet d'éviter que le gaz contenu dans les bulles de gaz formées dans un groupe de plans de découpe verticaux ne se propage vers les bulles de gaz formées dans un groupe adjacent de plans de découpe verticaux plus superficiel, comme il ressortira de la description qui va suivre.

La modulation optique de phase est réalisée au moyen d'un masque de phase. L'énergie du faisceau laser incident est conservée après modulation, et la mise en forme du faisceau est réalisée en agissant sur son front d'onde. La phase d'une onde électromagnétique représente la situation instantanée de l'amplitude d'une onde électromagnétique. La phase dépend aussi bien du temps que de l'espace. Dans le cas de la mise en forme spatiale d'un faisceau laser, seules les variations dans l'espace de la phase sont considérées.

Le front d'onde est défini comme la surface des points d'un faisceau possédant une phase équivalente (i.e. la surface constituée des points dont les temps de parcours depuis la source ayant émis le faisceau sont égaux). La modification de la phase spatiale d'un faisceau passe donc par la modification de son front d'onde.

Dans le cadre de la présente invention, la modulation de phase du front d'onde permet de générer un unique faisceau laser modulé qui forme :
- soit plusieurs points d'impact seulement dans le plan de découpe (utilisés pour former un plan de découpe horizontal) ; dans ce cas, le faisceau laser modulé est unique tout au long du chemin de propagation, la modulation de phase du front d'onde permettant de retarder ou d'avancer la phase des différents points de la surface du faisceau par rapport au front d'onde initial afin que chacun de ces points réalisent une interférence constructive en N points distincts dans le plan focal d'une lentille, la redistribution d'énergie en une pluralité de points d'impact n'ayant lieu que dans un seul plan (i.e. le plan de focalisation) et pas tout au long du chemin de propagation du faisceau laser modulé.,
- soit une ligne d'impact perpendiculaire au plan focal de l'appareil de découpe (utilisée pour former un plan de découpe vertical).

Des aspects préférés mais non limitatifs de l'appareil de découpe sont les suivants :
- la deuxième pluralité de plans de découpe verticaux peut être décalée latéralement par rapport à la première pluralité de plans de découpe verticaux d'une distance comprise entre 5µm et 500 µm ;
- la deuxième pluralité de plans de découpe verticaux peut être décalée latéralement par rapport à la première pluralité de plans de découpe verticaux selon des premier et deuxième axes perpendiculaires à l'axe optique, les premier et deuxième axes étant orthogonaux entre eux ;
- pour la réalisation de chaque plan de découpe horizontal, l'unité de commande peut être configurée pour :
   o appliquer un masque de phase multipoints au système de mise en forme pour produire un unique faisceau laser modulé multipoints, le masque de phase multipoints étant calculé pour répartir l'énergie du faisceau laser modulé multipoints en au moins deux points d'impact dans le plan focal de l'appareil de découpe,
   o commander le déplacement du système de focalisation pour faire coïncider le plan focal de l'appareil de découpe à la profondeur souhaitée pour le plan de découpe horizontal,
   o piloter le scanner optique de balayage pour déplacer les points d'impact de l'unique faisceau laser modulé multipoints le long d'un premier chemin de déplacement,
   o activer la source laser femtoseconde ;
- pour la réalisation de chaque plan de découpe vertical de la première pluralité, l'unité de commande peut être configurée pour :
   o appliquer au système de mise en forme, une consigne de modulation axiconique afin de produire un faisceau laser modulé de type Bessel à partir du faisceau laser gaussien, ladite consigne de modulation comportant un masque de phase émulant un axicon appliqué sur le modulateur spatial de lumière, ledit faisceau laser modulé de type Bessel créant une ligne d'impact permettant de générer une bulle de gaz oblongue dans le tissu,
   o piloter le scanner optique de balayage pour déplacer la ligne d'impact du faisceau laser modulé de type Bessel le long d'un deuxième chemin optique de déplacement pour former un ensemble de bulles de gaz oblongues adjacentes ;
- pour la réalisation de chaque plan de découpe vertical de la deuxième pluralité, l'unité de commande est configurée pour :
   o appliquer au système de mise en forme la consigne de modulation axiconique,
   o piloter le scanner optique de balayage pour déplacer la ligne d'impact du faisceau laser modulé de type Bessel le long d'un troisième chemin optique décalé latéralement relativement au deuxième chemin optique ;
- chaque plan de découpe vertical est composé d'un empilement de plusieurs ensembles de bulles de gaz oblongues adjacentes, l'unité de commande (60) étant configurée pour :
   o piloter le système optique de focalisation afin de positionner le plan focal de l'appareil de découpe à une distance prédéfinie non nulle du premier plan de découpe horizontal, ladite distance prédéfinie étant inférieure à la longueur de la ligne d'impact du faisceau laser modulé de type Bessel de sorte que la ligne d'impact intersecte partiellement le premier plan de découpe horizontal,
   o piloter le scanner optique de balayage pour déplacer la ligne d'impact du faisceau laser modulé de type Bessel pour former un premier ensemble de bulles de gaz oblongues adjacentes,
   o piloter le système optique de focalisation afin de positionner le plan focal de l'appareil de découpe à la distance prédéfinie du premier ensemble de bulles de gaz oblongues adjacentes de sorte que la ligne d'impact intersecte partiellement le premier ensemble de bulles de gaz oblongues adjacentes,
   o piloter le scanner optique de balayage pour déplacer la ligne d'impact du faisceau laser modulé de type Bessel pour former un deuxième ensemble de bulles de gaz oblongues adjacentes ;
- la distance prédéfinie peut être comprise en un $\frac{1}{5}$ et ⅓ de la longueur de la ligne d'impact. L'invention concerne également un procédé de découpe d'un tissu, par exemple un tissu humain ou animal préalablement prélevé, à partir d'un appareil de découpe incluant :
- une source laser femtoseconde configurée pour émettre un faisceau laser gaussien sous forme d'impulsions,
- un système de mise en forme en aval de la source laser femtoseconde et positionné sur la trajectoire du faisceau laser gaussien, pour moduler la phase du front d'onde du faisceau laser gaussien, le système de mise en forme comprenant un modulateur spatial de lumière et étant configuré pour produire un faisceau laser modulé à partir du faisceau laser gaussien,
- un scanner optique de balayage disposé en aval du système de mise en forme pour déplacer le faisceau laser modulé,
- un système optique de focalisation en aval du système de mise en forme, pour focaliser le faisceau laser modulé dans un plan focal de l'appareil de découpe et pour déplacer le plan focal de l'appareil de découpe en une pluralité de positions le long d'un axe optique de propagation du faisceau laser modulé,
***remarquable en ce que*** le procédé de découpe comprend une phase de réalisation de plans de découpe horizontaux et verticaux successifs en pilotant la source laser femtoseconde, le système de mise en forme, le scanner optique de balayage, et le système optique de focalisation, les plans de découpe horizontaux s'étendant perpendiculairement à l'axe optique et les plans de découpe verticaux s'étendant parallèlement à l'axe optique, ladite phase de réalisation comprenant les étapes consistant à :
- former un premier plan de découpe horizontal,
- former, au-dessus du premier plan de découpe horizontal, une première pluralité de plans de découpe verticaux,
- former, au-dessus de la première pluralité de plans de découpe verticaux, un deuxième plan de découpe horizontal, le premier plan de découpe horizontal étant plus profond dans le tissu que le deuxième plan de découpe horizontal,
- former, au-dessus du deuxième plan horizontal, une deuxième pluralité de plans verticaux décalée latéralement relativement à la première pluralité de plans verticaux.

Des aspects préférés mais non limitatifs du procédé selon l'invention sont les suivants :
- la deuxième pluralité de plans de découpe verticaux peut être décalée latéralement par rapport à la première pluralité de plans de découpe verticaux d'une distance comprise entre 5µm et 500 µm ;
- la deuxième pluralité de plans de découpe verticaux peut être décalée latéralement par rapport à la première pluralité de plans de découpe verticaux selon des premier et deuxième axes perpendiculaires à l'axe optique, les premier et deuxième axes étant orthogonaux entre eux ;
- chaque étape consistant à former un plan de découpe horizontal peut comprendre les sous-étapes suivantes :
   o application d'un masque de phase multipoints au système de mise en forme pour produire un unique faisceau laser modulé multipoints, le masque de phase multipoints étant calculé pour répartir l'énergie du faisceau laser modulé multipoints en au moins deux points d'impact dans le plan focal de l'appareil de découpe,
   o déplacement par le système de focalisation du plan focal de l'appareil de découpe à la profondeur souhaitée pour le plan de découpe horizontal,
   o déplacement par le scanner optique de balayage des points d'impact de l'unique faisceau laser modulé multipoints le long d'un premier chemin de déplacement, et
   o émission d'un faisceau laser gaussien par la source laser femtoseconde ;
- chaque étape consistant à former un plan de découpe vertical de la première pluralité peut comprendre les sous-étapes suivantes :
   o application d'une consigne de modulation axiconique au système de mise en forme afin de produire un faisceau laser modulé de type Bessel, ladite consigne de modulation comportant un masque de phase émulant un axicon appliqué sur le modulateur spatial de lumière, ledit masque de phase ayant une symétrie de révolution autour d'un point central de symétrie, le niveau de gris de chaque point du masque de phase variant en fonction de la distance entre ledit point et le point central de symétrie, ledit faisceau laser modulé de type Bessel créant une ligne d'impact permettant de générer une bulle de gaz oblongue dans le tissu,
   o déplacement par le scanner optique de balayage de la ligne d'impact du faisceau laser modulé de type Bessel le long d'un deuxième chemin optique de déplacement pour former un ensemble de bulles de gaz oblongues adjacentes ;
- chaque étape consistant à former un plan de découpe vertical de la deuxième pluralité peut comprendre les sous-étapes suivantes :
   o application de la consigne de modulation axiconique au système de mise en forme,
   o déplacement par le scanner optique de balayage de la ligne d'impact du faisceau laser modulé de type Bessel le long d'un troisième chemin optique de déplacement décalé latéralement relativement au deuxième chemin optique ;
- chaque plan de découpe vertical peut être composé d'un empilement de plusieurs ensembles de bulles de gaz oblongues adjacentes, chaque étape consistant à former un plan de découpe vertical comprenant les sous-étapes suivantes :
   o déplacement par le système optique de focalisation du plan focal de l'appareil de découpe à une distance prédéfinie non nulle du premier plan de découpe horizontal, ladite distance prédéfinie étant inférieure à la longueur de la ligne d'impact du faisceau laser modulé de type Bessel de sorte que la ligne d'impact intersecte partiellement le premier plan de découpe horizontal,
   o déplacement par le scanner optique de balayage de la ligne d'impact du faisceau laser modulé de type Bessel pour former un premier ensemble de bulles de gaz oblongues adjacentes,
   o déplacement par le système optique de focalisation du plan focal de l'appareil de découpe à la distance prédéfinie du premier ensemble de bulles de gaz oblongues adjacentes de sorte que la ligne d'impact intersecte partiellement le premier ensemble de bulles de gaz oblongues adjacentes,
   o déplacement par le scanner optique de balayage de la ligne d'impact du faisceau laser modulé de type Bessel pour former un deuxième ensemble de bulles de gaz oblongues adjacentes ;
- la distance prédéfinie peut être comprise en un $\frac{1}{5}$ et ⅓ de la longueur de la ligne d'impact.

### BREVE DESCRIPTION DES DESSINS

D'autres caractéristiques et avantages de l'invention ressortiront clairement de la description qui en est réalisée ci-après, à titre indicatif et nullement limitatif, en référence aux figures annexées, dans lesquelles :
- la figure 1 est une représentation schématique d'un œil d'un patient,
- la figure 2 est une représentation schématique de bulles de gaz crées pour former des cubes de tissu élémentaires à partir de plans de découpe horizontaux et verticaux,
- la figure 3 est une représentation schématique d'un empilement de plans de découpe horizontaux et verticaux obtenus grâce à l'appareil de découpe décrit dans WO 2016/055539,
- la figure 4 est une représentation schématique illustrant une accumulation de gaz suite à la formation de plans de découpe verticaux superposés à partir de l'appareil de découpe décrit dans WO 2016/055539,
- la figure 5 est une représentation schématique d'un appareil de découpe selon l'invention,
- la figure 6 est une représentation schématique illustrant la focalisation d'un faisceau non diffractant de type Bessel,
- la figure 7a est une image d'un premier masque de phase permettant d'émuler le comportement d'un axicon négatif sur un SLM de l'appareil de découpe selon l'invention,
- la figure 7b est une image d'un deuxième masque de phase permettant d'émuler le comportement d'un axicon positif sur le SLM de l'appareil de découpe selon l'invention,
- la figure 8 est un schéma de montage partiel du dispositif de découpe,
- la figure 9 est une représentation schématique d'un faisceau de Bessel,
- la figure 10 est une représentation schématique d'étapes de commande lors de la réalisation d'un plan de découpe horizontal,
- la figure 11 est une représentation schématique d'étapes de commande lors de la réalisation d'un plan de découpe vertical,
- la figure 12 est une représentation schématique illustrant la formation d'un plan de découpe vertical à partir d'un faisceau laser de Bessel,
- la figure 13 est une représentation schématique illustrant un ensemble de lignes d'impact utilisées pour réaliser des pans de bulles de gaz oblongues empilés,
- la figure 14 est une représentation schématique d'étapes de commande lors de la réalisation de plans de découpe horizontaux et verticaux successifs,
- la figure 15 est une représentation schématique de plans de découpe horizontaux et verticaux réalisés successivement.

### EXPOSE DETAILLE DE L'INVENTION

L'invention concerne un système de découpe d'un tissu humain au moyen d'un laser femtoseconde. Dans la suite de la description, l'invention sera décrite, à titre d'exemple, pour la découpe d'un cristallin d'un œil humain ou animal.

### 1. Appareil de découpe

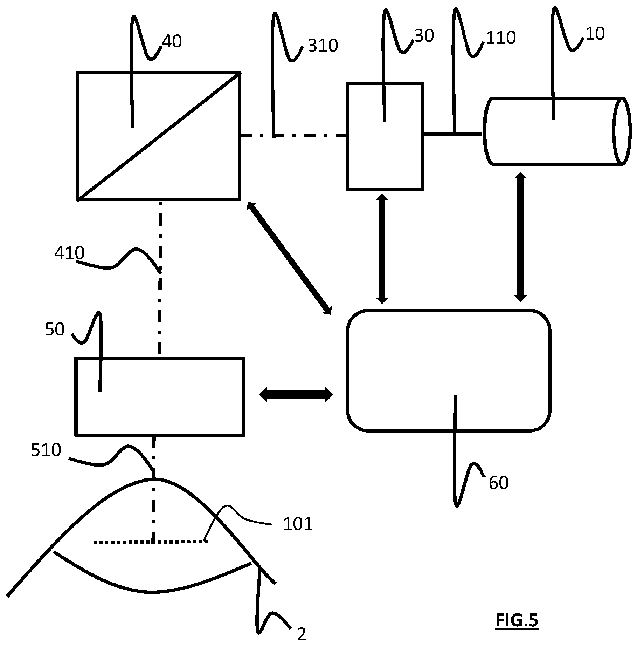

En référence à la figure 5, on a illustré un mode de réalisation de l'appareil de découpe selon l'invention. Celui-ci peut être disposé entre une source laser femtoseconde 10 et une cible à traiter 2.

La source laser femtoseconde 10 est apte à émettre un faisceau laser gaussien sous la forme d'impulsions. A titre d'exemple, la source laser femtoseconde 10 émet une lumière de 1030 nm de longueur d'onde, sous la forme d'impulsions de 400 femtosecondes. La source laser femtoseconde 10 possède une puissance de 20W et une fréquence de 500 kHz.

La cible 2 est par exemple un tissu humain ou animal à découper tel qu'une cornée ou un cristallin.

L'appareil de découpe comprend :
- un système de mise en forme 30 positionné sur la trajectoire du faisceau laser 110 issu du laser femtoseconde 10,
- un scanner optique de balayage 40 en aval du système de mise en forme 30,
- un système optique de focalisation 50 en aval du scanner optique de balayage 40, et
- une unité de commande 60.

Le système de mise en forme 30 permet de moduler la phase du faisceau laser 110 issu de la source laser femtoseconde 10. Ce système de mise en forme 30 est avantageusement un composant programmable.

Le scanner optique de balayage 40 permet d'orienter le faisceau laser modulé en phase 310 issu du système de mise en forme 30 pour déplacer le motif de découpe le long d'un chemin de déplacement prédéfini par l'utilisateur dans le plan focal 101 du système de découpe.

Le système optique de focalisation 50 permet de déplacer le plan focal 101 - correspondant au plan de découpe - du faisceau laser modulé et dévié 410.

L'unité de commande 60 permet de piloter le système de mise en forme 30, le scanner optique de balayage 40 et le système optique de focalisation 50.

Cet appareil de découpe est adapté pour former des plans de découpe horizontaux et verticaux. En fonction du type de plan de découpe souhaité (vertical ou horizontal), l'unité de commande 60 :
- configure le système de mise en forme pour moduler le faisceau laser 110 en fonction de l'aspect désiré pour les points/lignes d'impact, et
- contrôle le scanner optique de balayage 40 et du système optique de focalisation 50 pour générer le plan de découpe souhaité.

Comme il sera décrit plus en détails dans la suite, les inventeurs ont développé une solution de configuration originale de l'appareil de découpe pour la formation de plans de découpe verticaux.

### 2. Eléments de l'appareil de découpe

### 2.1. Système de mise en forme

Le système de mise en forme spatiale 30 du faisceau laser permet de faire varier la surface d'onde du faisceau laser 110 en fonction de la forme souhaitée :
- pour le (ou les) point(s) d'impact du faisceau laser modulé dans le cas de la formation d'un plan de découpe horizontal, ou
- pour la (ou les) ligne(s) d'impact du faisceau laser modulé dans le cas de la formation d'un plan de découpe vertical.

Le système de mise en forme 30 comprend de préférence un modulateur spatial de lumière, connu sous le sigle SLM, de l'acronyme anglais *« Spatial Light Modulator ».*

Le SLM permet de moduler la répartition finale d'énergie du faisceau laser 110 issu de la source laser 10. Le SLM est un dispositif constitué d'une couche de cristaux liquides à orientation contrôlée permettant de façonner d'une manière dynamique le front d'onde, et donc la phase du faisceau laser 110. La couche de cristaux liquides d'un SLM est organisée comme une grille (ou matrice) de pixels. L'épaisseur optique de chaque pixel est contrôlée électriquement par orientation des molécules de cristal liquide appartenant à la surface correspondant au pixel. Le SLM exploite le principe d'anisotropie des cristaux liquides, c'est-à-dire la modification de l'indice des cristaux liquides, en fonction de leur orientation spatiale. L'orientation des cristaux liquides peut être réalisée à l'aide d'un champ électrique. Ainsi, la modification de l'indice des cristaux liquides modifie le front d'onde du faisceau laser.

D'une manière connue, le SLM met en œuvre un masque de phase, c'est-à-dire une carte déterminant comment la phase du faisceau laser 110 doit être modifiée pour obtenir une répartition d'amplitude donnée. Le masque de phase est une image bidimensionnelle dont chaque point est associé à un pixel respectif du SLM. Ce masque de phase permet de piloter l'indice de chaque cristal liquide du SLM en convertissant la valeur associée à chaque point du masque - représentée en niveaux de gris compris entre 0 et 255 (donc du noir au blanc) - en une valeur de commande - représentée en une phase comprise entre 0 et 2π. Ainsi, le masque de phase est une consigne de modulation affichée sur le SLM pour entraîner en réflexion un déphasage spatial inégal du faisceau laser 110 illuminant le SLM. Bien entendu, l'homme du métier appréciera que la plage de niveau de gris puisse varier en fonction du modèle de SLM utilisé. Par exemple dans certains cas, la plage de niveau de gris peut être comprise entre 0 et 220.

Différents masques de phase peuvent être appliqués au SLM en fonction du type de plan de découpe que souhaite réaliser l'utilisateur, à savoir :
- soit un plan de découpe vertical,
- soit un plan de découpe horizontal.

Pour la réalisation d'un plan de découpe vertical, le masque de phase utilisé (ci-après dénommé *« masque de phase conique* ») permet d'appliquer une modulation de phase linéaire avec symétrie de rotation. On obtient ainsi un faisceau laser modulé de type Bessel. Pour la réalisation d'un plan de découpe horizontal, le masque de phase utilisé (ci-après dénommé *« masque de phase multipoints* ») permet d'appliquer une modulation de phase pour répartir l'énergie du faisceau laser en au moins deux points d'impact formant un motif dans le plan focal du système de découpe. On obtient ainsi un faisceau laser modulé de type multipoints.

### 2.1.1. Plan de découpe vertical

En ce qui concerne la découpe d'un plan vertical, les inventeurs proposent de moduler la phase du faisceau laser 110 issu de la source laser femtoseconde 10 de sorte à produire, en aval du système de mise en forme 30, un faisceau laser modulé 310 de type Bessel.

Un faisceau de Bessel est dit *« non diffractant* » car il possède la propriété de garder un profil constant le long de l'axe optique de propagation du faisceau laser (ci-après dénommé « *axe optique* »), contrairement au comportement d'un faisceau laser Gaussien (tel que le faisceau laser 110 issu de la source laser femtoseconde 10) qui se disperse lorsqu'il est focalisé.

### 2.1.1.1. Faisceau de Bessel

Un faisceau de Bessel d'ordre zéro parfait peut être défini mathématiquement comme un faisceau dont le champ électrique € est formellement décrit par la fonction de Bessel d'ordre zéro de première espèce J₀ : $E \left(r, ϕ, z\right) {A}_{0} {J}_{0} \left({k}_{r}r\right) e jk z z$ où :
- A₀ est l'amplitude du champ électrique,
- k_{z} et kᵣ sont les vecteurs d'onde longitudinal et radial,
- z, r, et ϕ sont les composantes longitudinale, radiale, et azimutale.

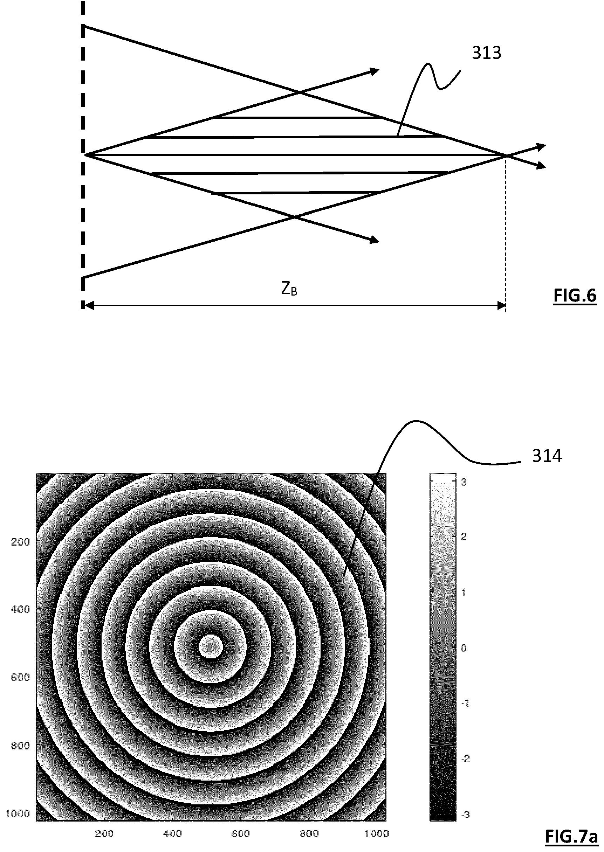

En référence à la figure 6, la formation du faisceau de Bessel 313 résulte de l'interférence d'ondes planes dont les vecteurs d'onde forment une surface conique.

En théorie, l'extension transverse de la structure annulaire est infinie, ainsi que la distance de propagation non-diffractive.

En pratique, le faisceau de Bessel expérimental présente une distance de propagation non-diffractive finie Z_{B} le long de l'axe optique du fait de la propagation finie observée en optique et de la quantité limitée d'énergie. Cette distance finie Z_{B} de propagation non diffractive définit une zone de non-diffraction ZND.

Il est admis que Z_{B} >> Z_{R}, Z_{R} étant la distance de Rayleigh du faisceau gaussien usuel de taille transverse similaire. En d'autres termes, la profondeur (i.e. dimension selon une direction parallèle à l'axe optique de propagation du faisceau laser) de chaque point d'impact d'un faisceau de Bessel est beaucoup plus grande que la profondeur de chaque point d'impact avec un faisceau laser gaussien (tel que le faisceau laser issu de la source laser femtoseconde).

Ainsi, l'utilisation d'un faisceau de Bessel permet de découper une profondeur de tissu beaucoup plus importante qu'avec un faisceau Gaussien. En particulier, à partir d'une seule ligne d'impact d'un faisceau de Bessel, il est possible de découper un tissu sur une profondeur équivalente à celle de quatre points d'impact superposés d'un faisceau gaussien. Le déplacement, par le scanner optique de balayage, d'une ligne d'impact d'un faisceau de Bessel permet de générer un plan de découpe vertical parfaitement vertical quatre fois plus rapidement qu'avec un point d'impact de faisceau gaussien.

De par sa formation spécifique basée sur un front d'onde conique, le faisceau de Bessel présente des propriétés d'auto régénération remarquables, ce qui signifie que le faisceau peut se régénérer lui-même au sein de la zone de non-diffraction ZND après n'importe quel obstacle sur son chemin. Ceci permet d'assurer la qualité de la découpe des plans verticaux en garantissant la formation d'une bulle de gaz étendue à chaque tir de la source laser 10, même lorsqu'une partie du faisceau laser modulé 310 est masquée par un obstacle.

### 2.1.1.2. Masque de phase conique pour former un faisceau laser modulé de type Bessel

Il existe différentes techniques pour générer un faisceau de Bessel à partir d'un faisceau laser gaussien. Ces techniques impliquent généralement une modulation de phase axiconique.

En particulier, le faisceau de Bessel peut être obtenu en utilisant une lentille conique connue sous le nom *« d'axicon ».* La lentille conique peut être concave/creux (on parle *« d'axicon négatif* ») ou convexe/bombé (on parle *« d'axicon positif »*)*.*

Les inventeurs proposent quant à eux d'utiliser le système de mise en forme 30 incluant le SLM pour générer le faisceau de Bessel afin d'éviter l'utilisation d'un élément optique/mécanique. A cet effet, un masque de phase conique (permettant d'émuler un axicon) est appliqué au SLM par l'unité de commande 60. Le SLM permet alors une modulation de phase conique du faisceau laser gaussien 110 issu de la source laser femtoseconde 10. Ainsi en utilisant le même SLM, il devient possible de réaliser un plan de découpe horizontal en multipoints, puis des plans de découpe verticaux en modalité faisceau de Bessel sans changer d'éléments optiques et donc en réduisant considérablement le temps de la procédure chirurgicale à un temps compris entre 30 secondes et 1 minute, compatible avec une application sur le globe oculaire du patient de moins de 3 minutes.

Le masque de phase conique à appliquer au SLM du système de mise en forme pour former un faisceau laser modulé de Bessel peut être calculé :
- en utilisant un algorithme de partition (Vellekoop et Mosk, 2008),
- ou tout autre algorithme connu de l'homme du métier.

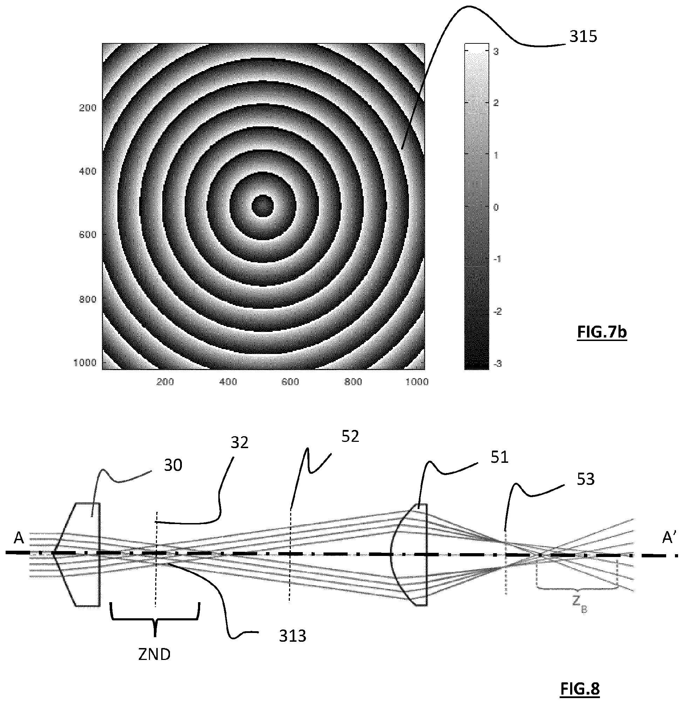

Deux exemples de tels masques de phase sont illustrés aux figures 7a et 7b. Lorsque l'un des premier et deuxième masques de phase est appliqué sur le SLM, le SLM est capable d'imprimer le profil de phase d'un axicon sur le faisceau laser gaussien 110 d'entrée pour obtenir un faisceau laser modulé de type Bessel 310 en sortie du système de mise en forme 30.

En référence à la figure 7a, le premier masque de phase conique (référencé 314) permet d'émuler le comportement d'un axicon négatif (i.e. axicon concave). En référence à la figure 7b, le deuxième masque de phase conique (référencé 315) permet d'émuler le comportement d'un axicon positif (i.e. axicon convexe). Ces premier et deuxième masques de phase présentent chacun une symétrie de révolution autour d'un point central de symétrie, le niveau de gris de chaque pixel variant en fonction de la distance entre ledit pixel et le point central de symétrie.

Lorsque l'un des masques de phase illustrés aux figures 7a et 7b est appliqué sur le SLM, le système de mise en forme 30 permet de former un faisceau laser modulé de type Bessel 310 (en sortie du système de mise en forme 30) à partir du faisceau laser gaussien 110 issu de la source laser femtoseconde 10 (en entrée du système de mise en forme 30). On obtient ainsi un faisceau laser modulé présentant une distribution spatiale d'intensité en faisceau de Bessel

### 2.1.1.3. Montage de l'appareil de découpe dans le cadre de la découpe d'un tissu à partir d'un faisceau laser modulé de type Bessel

On a illustré à la figure 8 un schéma de montage de l'appareil de découpe. Ce schéma de montage est partiel en ce qu'il ne fait pas apparaître la source laser femtoseconde, et le scanner optique de balayage. Par ailleurs dans cette figure 8, le système optique de focalisation 50 (dans son ensemble) est représenté par une lentille équivalente 51, étant bien entendu pour l'homme du métier que le système optique de focalisation 50 ne consiste pas uniquement en une lentille fixe.

En référence à la figure 8, le faisceau de Bessel 313 est formé juste après le plan de modulation de phase conique, c'est-à-dire juste après le SLM du système de mise en forme 30. Le SLM simulant une lentille conique (axicon négatif ou positif), la tache centrale d'intensité maximale du faisceau de Bessel se forme dans le plan focal image 32 du SLM.

La lentille équivalente 51 du système optique de focalisation 50 est disposé en aval du système de mise en forme 30, et est agencée de sorte que le plan focal objet 52 de la lentille équivalente s'étend à une distance non nulle du plan focal image 32 du système de mise en forme 30 long de l'axe optique.

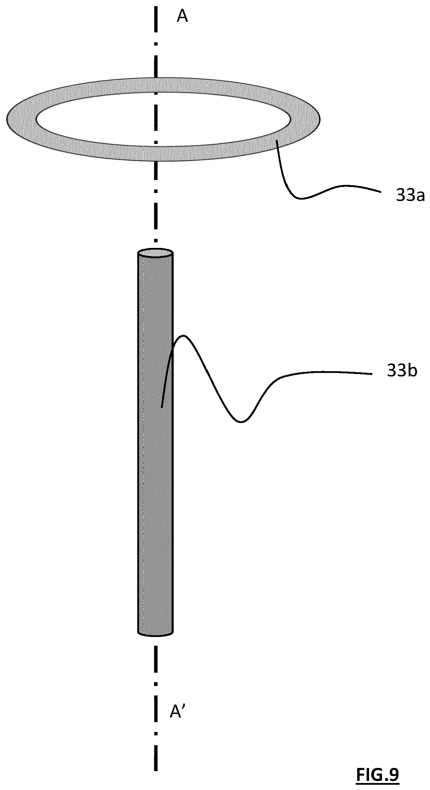

Ainsi, le plan focal objet 52 de la lentille équivalente 51 du système optique de focalisation 50 s'étend en dehors de la zone de non-diffraction ZND du faisceau de Bessel, de sorte qu'en sortie du système de découpe, une ligne d'impact tel qu'illustré à la figure 9 est obtenue. Plus précisément, le faisceau de Bessel est composé :
- d'un anneau de Bessel 33a focalisé au plan focal image 53 de la lentille équivalente 51 (correspondant au plan focal de l'appareil de découpe),
- d'une ligne 33b de concentration des rayons du faisceau de Bessel - correspondant à l'image de la zone de non-diffraction ZND - ladite ligne 33b se formant en dehors du plan focal image 53 de la lentille équivalente 51.

Dans le cadre de la présente invention, c'est la ligne 33b qui constitue la ligne d'impact utilisée pour réaliser le plan de découpe vertical (l'énergie contenu dans l'anneau de Bessel n'est pas suffisante pour former une bulle de gaz).

La ligne 33b peut se former soit avant, soit après l'anneau 33a, en fonction du signe de la modulation de phase. En d'autres termes la position de la ligne 33b relativement à l'anneau 33a dépend du type d'axicon (positif ou négatif) émulé grâce au masque de phase conique. Comme la zone de non-diffraction ZND de Bessel (i.e. la ligne 33b) est déplacée en dehors du plan focal du système de découpe, aucune interférence ne se produit avec la lumière non modulée. Ceci permet un meilleur contrôle du profil d'intensité sans perte d'énergie lié au filtrage du faisceau.

### 2.1.2. Plan de découpe horizontal

En ce qui concerne la découpe d'un plan horizontal, les inventeurs proposent de moduler la phase du faisceau laser 110 issu de la source laser femtoseconde 10 de sorte à produire, en aval du système de mise en forme 30, un faisceau laser modulé de type multipoints.

A cet effet, un masque de phase multipoints à appliquer au SLM pour obtenir le faisceau laser modulé multipoints est calculé. Le masque de phase multipoints est généralement calculé par :
- un algorithme itératif basé sur la transformée de Fourier, tel qu'un algorithme de type *« IFTA* », acronyme de l'expression anglo-saxonne « *Iterative Fourrier Transform Algorithm* », ou par
- divers algorithmes d'optimisation, tels que des algorithmes génétiques, ou le recuit simulé.

Ce masque de phase multipoints est calculé pour former des pics d'intensité dans le plan focal de l'appareil de découpe, chaque pic d'intensité produisant un point d'impact respectif dans le plan focal de l'appareil de découpe.

Plus précisément, le masque de phase multipoints est calculé pour distribuer l'énergie du faisceau laser issu de la source laser en plusieurs points d'impact - formant un motif - dans le plan focal de l'appareil de découpe. Cette modulation du front d'onde peut être vue comme un phénomène d'interférences en deux dimensions. Chaque portion du faisceau laser initial issu de la source est retardée ou avancée par rapport au front d'onde initial afin que chacune de ces portions soit redirigée de façon à réaliser une interférence constructive en N points distincts dans le plan focal d'une lentille. Cette redistribution d'énergie en une pluralité de points d'impact n'a lieu que dans un seul plan (i.e. le plan de focalisation) et pas tout au long du chemin de propagation du faisceau laser modulé. Ainsi, le faisceau laser multipoints obtenu (en sortie du système de mise en forme 30) est unique : l'observation du faisceau laser modulé avant ou après le plan focal de l'appareil de découpe (correspondant au plan focal du système optique de focalisation 50) ne permet pas d'identifier une redistribution de l'énergie en une pluralité de points d'impact distincts, du fait de ce phénomène qu'on peut assimiler à des interférences constructives (qui n'ont lieu que dans un plan et pas tout au long de la propagation comme dans le cas de la séparation d'un faisceau laser initial en une pluralité de faisceaux laser secondaires).

Le fait de disposer d'un unique faisceau laser modulé multipoint facilite l'intégration d'un système de balayage - tel qu'un scanner optique - pour déplacer la pluralité de points d'impact dans le plan focal. En effet, le diamètre d'entrée d'un système de balayage étant de l'ordre du diamètre du faisceau laser initial issu de la source laser 10, l'utilisation d'un unique faisceau laser modulé multipoints (dont le diamètre est sensiblement égal au diamètre du faisceau laser initial) limite les risques d'aberration qui peuvent se produire avec la technique de subdivision de faisceau telle que décrite dans US 2010/0133246.

Le système de mise en forme 30 permet donc, à partir d'un faisceau laser gaussien générant un unique point d'impact, et au moyen du masque de phase multipoints appliqué au SLM, de répartir son énergie par modulation de phase de sorte à générer simultanément plusieurs points d'impact dans le plan focal de l'appareil de découpe, à partir d'un unique faisceau laser mis en forme par modulation de phase (un seul faisceau en amont et en aval du SLM). Ceci permet de diminuer le temps nécessaire pour réaliser un plan de découpe horizontal.

Par exemple dans le cas d'un faisceau laser modulé multipoints ayant trois points d'impact, le temps nécessaire à la réalisation d'un plan de découpe horizontal est réduit d'un facteur six (par rapport à la réalisation d'u même plan de découpe horizontal en utilisant un faisceau laser gaussien générant un unique point d'impact). L'homme du métier sait calculer une valeur en chaque point du masque de phase multipoints pour distribuer l'énergie du faisceau laser en différents points d'impact dans le plan focal de l'appareil de découpe.

### 2.2. Scanner optique de balayage

Le scanner optique de balayage 40 permet de dévier le faisceau laser modulé (de Bessel ou multipoints) 310 de sorte à déplacer :
- pour la réalisation d'un plan de découpe horizontal, le (ou les) point(s) d'impact en une pluralité de positions le long d'un premier chemin de déplacement,
- pour la réalisation d'un plan de découpe vertical, la (ou les) ligne(s) d'impact en une pluralité de positions le long d'un deuxième chemin de déplacement.

Le scanner optique de balayage 40 comprend :
- un orifice d'entrée pour recevoir le faisceau laser modulé en phase 31 issu de l'unité de mise en forme 30,
- un (ou plusieurs) miroir(s) optique(s) pivotant autour d'au moins deux axes pour dévier le faisceau laser modulé en phase 310, et
- un orifice de sortie pour envoyer le faisceau laser modulé dévié 410 vers le système optique de focalisation 50.

Le scanner optique 40 utilisé est par exemple une tête de balayage IntelliScan III de la société SCANLAB AG.

Les orifices d'entrée et de sortie d'un tel scanner optique 40 présentent un diamètre de l'ordre de 10 à 20 millimètres, et les vitesses de balayage atteignables sont de l'ordre de 1m/s à 10m/s.

Le (ou les) miroir(s) est (sont) connecté(s) à un (ou des) moteur(s) pour permettre leur pivotement. Ce(s) moteur(s) pour le pivotement du (ou des) miroir(s) est (sont) avantageusement piloté(s) par l'unité de l'unité de commande 60 qui sera décrite plus en détails dans la suite.

L'unité de commande 60 est programmée pour piloter le scanner optique de balayage 40 de sorte à déplacer :
- le (ou les) point(s) d'impact le long du premier chemin de déplacement,
- la (ou les) lignes d'impact le long du deuxième chemin de déplacement.

Dans le cas d'un plan de découpe horizontal, le premier chemin de déplacement comprend une pluralité de segments de découpe. Le premier chemin de déplacement peut avantageusement présenter une forme de créneau.

Dans le cas d'un plan de découpe vertical, le deuxième chemin de déplacement comprend un segment. L'unité de commande 60 peut être configurée pour commander au scanner optique 40 un déplacement en va et vient de la ligne d'impact de Bessel le long dudit segment pour découper le plan de découpe vertical sur toute sa profondeur. Par exemple, si le scanner optique 40 débute le segment par la gauche, il débutera ce segment par la droite au retour, puis par la gauche, puis par la droite et ainsi de suite sur toute la hauteur du plan de découpe vertical.

Le balayage du faisceau a une influence sur le résultat de la découpe obtenue. En effet, la vitesse de balayage utilisée, ainsi que le pas du balayage, sont des paramètres influençant la qualité de la découpe.

Avantageusement, l'unité de commande 60 peut être programmée pour activer le laser femtoseconde 10 lorsque la vitesse de balayage du scanner optique 40 est supérieure à une valeur seuil. Ceci permet de synchroniser l'émission du faisceau laser 110 avec le balayage du scanner optique de balayage 40. Plus précisément, l'unité de commande 60 active le laser femtoseconde 10 lorsque la vitesse de pivotement du (ou des) miroir(s) du scanner optique 40 est constante. Ceci permet d'améliorer la qualité de découpe par la réalisation d'un surfaçage homogène du plan de découpe.

### 2.3. Système optique de focalisation

Le système optique de focalisation 50 permet de déplacer le plan focal de l'appareil de découpe en fonction du type de plan de découpe à réaliser.

Le système optique de focalisation 50 comprend :
- un orifice d'entrée pour recevoir le faisceau laser modulé en phase et dévié issu du scanner optique de balayage 40,
- une (ou plusieurs) lentille(s) motorisée(s) pour permettre son (leur) déplacement en translation le long du chemin optique du faisceau laser modulé et dévié, et
- un orifice de sortie pour envoyer le faisceau laser focalisé vers le tissu à traiter.

L'unité de commande 60 est programmée pour piloter le déplacement de la (ou des) lentille(s) du système optique de focalisation 50 de sorte à déplacer le plan focal de l'appareil de découpe en fonction du type de plan de découpe à réaliser.

Dans le cas d'un plan de découpe horizontal, le plan de découpe correspond au plan focal de l'appareil de découpe. L'unité de commande 60 pilote le déplacement de la (ou des) lentille(s) du système optique de focalisation 50 pour focaliser le faisceau laser modulé et dévié 410 à une profondeur désirée correspondant à la profondeur du plan de découpe à réaliser.

Dans le cas d'un plan de découpe vertical, le plan de découpe peut être situé :
- au-dessous du plan focal de l'appareil de découpe dans le cas où le masque de phase conique utilisé permet au SLM d'émuler un axicon positif (l'anneau de Bessel 33a est situé au-dessus de la ligne d'impact utilisée pour réaliser la découpe),
- au-dessus du plan focal de l'appareil de découpe dans le cas où le masque de phase conique utilisé permet au SLM d'émuler un axicon négatif (l'anneau de Bessel 33a est situé au-dessous de la ligne d'impact utilisée pour réaliser la découpe).

De préférence, la distance entre deux plans de découpe successifs est comprise entre 2 µm et 500 µm, et notamment :
- entre 2 et 20µm pour traiter un volume nécessitant une grande précision, par exemple en chirurgie réfractive, avec de préférence un espacement compris entre 5 et 10µm, ou
- entre 20 et 500µm pour traiter un volume ne nécessitant pas une grande précision, comme par exemple pour détruire la partie centrale d'un noyau cristallinien, avec de préférence un espacement compris entre 50 et 300µm.

Bien entendu, cette distance peut varier dans un volume composé d'un empilement de plans de découpe.

### 2.4. Unité de commande

Comme indiqué précédemment, l'unité de commande 60 permet de contrôler les différents composants de l'appareil de découpe, à savoir la source laser femtoseconde 10, le système de mise en forme 30, le scanner optique de balayage 40 et le système optique de focalisation 50.

L'unité de commande 60 est connectée à ces différents composants par l'intermédiaire d'un (ou plusieurs) bus de communication permettant :
- la transmission de signaux de commande tels que
   - le signal d'activation à la source laser femtoseconde 10,
   - le masque de phase au système de mise en forme 30,
   - la vitesse de balayage au scanner optique de balayage 40,
   - la position du scanner optique de balayage 40 le long du chemin de déplacement,
   - la profondeur de découpe au système optique de focalisation 50.
- la réception de données de mesure issues des différents éléments du système tels que
   - la vitesse de balayage atteinte par le scanner optique, ou
   - la position du système optique de focalisation, etc.

L'unité de commande 60 peut être composée d'une (ou plusieurs) station(s) de travail, et/ou d'un (ou plusieurs) ordinateur(s) ou peut être de tout autre type connu de l'homme du métier. L'unité de commande 60 peut par exemple comprendre un téléphone portable, une tablette électronique (tel qu'un IPAD^{®}), un assistant personnel (ou *« PDA* », sigle de l'expression anglo-saxonne *« Personal Digital Assistant* »)*,* etc.

Dans tous les cas, l'unité de commande 60 comprend un processeur programmé pour permettre le pilotage de la source laser femtoseconde 10, du système de mise en forme 30, du scanner optique de balayage 40, du système optique de focalisation 50, etc. Avantageusement, l'unité de commande 60 est programmée pour faire varier la forme du faisceau laser modulé entre deux plans de découpe successifs, notamment entre un plan de découpe horizontal et un plan de découpe vertical.

### 2.5. Principe de fonctionnement

On va maintenant décrire plus en détail le principe de fonctionnement du dispositif de découpe selon l'invention en détaillant :
- le fonctionnement de l'appareil pour réaliser un plan de découpe horizontal,
- le fonctionnement de l'appareil pour réaliser un plan de découpe vertical,
- le fonctionnement de l'appareil pour réaliser une superposition de plans de découpe horizontaux et verticaux empilés.

### 2.5.1. Formation d'un plan de découpe horizontal

Dans le cadre de la présente invention, la formation d'un plan de découpe horizontal est réalisée comme suit.

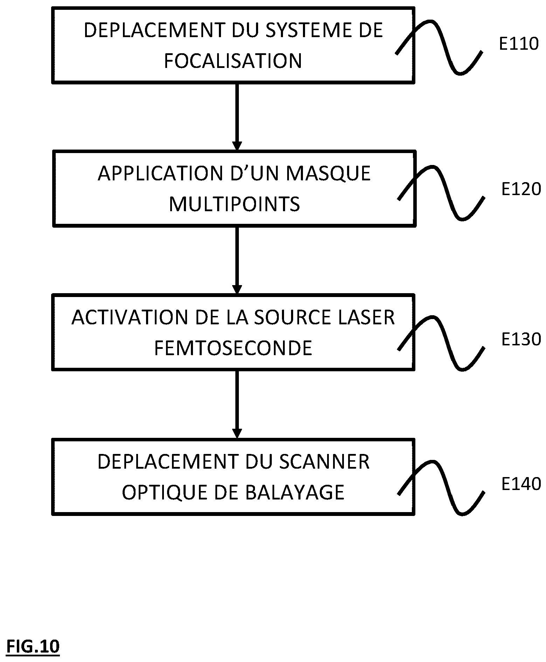

En référence à la figure 10, l'unité de commande 60 émet un signal de commande au système optique de focalisation 50 pour piloter son déplacement de sorte à faire coïncider le plan focal de l'appareil de découpe avec le plan de découpe horizontal souhaité (étape E110).

L'unité de commande 60 transmet un masque de phase multipoints au système de mise en forme 30 pour produire un faisceau laser modulé multipoints (étape E120).

L'unité de commande 60 active également le déplacement du scanner optique de balayage 40 jusqu'à une position initiale du premier chemin optique de balayage. Le balayage se faisant en X, Y, le scanner est équipé d'un ou plusieurs miroir(s). Par exemple, dans le mode de réalisation illustré à la figure 5, le scanner optique de balayage 40 comporte un premier miroir X et un deuxième miroir Y dont le pivotement permet de déplacer le faisceau laser modulé le long du premier chemin de déplacement. En variante, le scanner peut être équipé d'un unique miroir configuré pour pivoter autour de deux axes distincts.

Lorsque :
- le système de focalisation 50 et le scanner optique 40 sont en position (i.e. le scanner a atteint une position cible de début de ligne), que
- le masque de phase multipoints est chargé dans le système de mise en forme 30, et que
- la vitesse de pivotement du (ou des) miroir(s) du scanner optique 40 est constante, l'unité de commande 60 active la source laser femtoseconde 10 pour émettre une impulsion laser (étape E130).

La source laser femtoseconde 10 génère un faisceau laser 110 qui traverse le système de mise en forme 30. Le système de mise en forme 30 module la phase du faisceau laser pour produire un unique faisceau laser modulé multipoints.

Le faisceau laser modulé multipoints 310 sort du système de mise en forme 30 et entre dans le scanner optique 40 qui dévie le faisceau laser modulé multipoints 310.

Le faisceau laser modulé et dévié 410 entre dans le système optique de focalisation 50 qui focalise le faisceau dans le plan focal de l'appareil de découpe. Dans le plan focal, la consigne de modulation (i.e. le masque de phase multipoints) appliquée au système de mise en forme 30 permet de répartir l'énergie en une pluralité de points d'impact. Cette pluralité de points d'impact générés simultanément forme le motif. Chaque point d'impact du motif produit simultanément une bulle de gaz.

Le laser femtoseconde 10 continue d'émettre d'autres impulsions sous forme de faisceau laser à une cadence déterminée. Entre chaque impulsion le (ou les) miroir(s) pivote(nt) d'un certain angle, ce qui a pour conséquence de déplacer le motif 8 et de produire de nouvelles bulles de gaz décalées par rapport aux précédentes le long du premier chemin optique (étape E140). Les opérations de pilotage de la source laser femtoseconde 10 et du scanner optique de balayage 40 sont répétées pour former le plan de découpe horizontal.

En faisant varier la vitesse de déplacement du (ou des) miroir(s) et/ou la cadence de génération des impulsions par la source laser femtoseconde 10, il est possible de faire varier la distance entre deux motifs successifs.

Une fois que la ligne de découpe a été complétée, l'unité de commande 60 désactive la source laser femtoseconde 10, et commande le déplacement du scanner optique 40 jusqu'à une position suivante de découpe en fonction du premier chemin de déplacement.

Lorsque le scanner optique 40 est en position et que le (ou les) miroir(s) a (ont) atteint sa (leur) vitesse de consigne constante, l'unité de commande 60 active à nouveau la source laser femtoseconde 10. Le faisceau laser 110 traverse le système de mise en forme 30, le scanner optique 40 et le système optique de focalisation 50. Une nouvelle ligne de bulles de gaz - parallèle à la précédente - se forme dans le plan de découpe.

Lorsque le scanner optique 40 a balayé toutes les positions du premier chemin de déplacement, le plan de découpe horizontal est finalisé.

En résumé, pour la réalisation d'un plan de découpe horizontal, l'unité de commande 60 est configurée pour :
- appliquer un masque de phase multipoints au système de mise en forme 30 pour produire un unique faisceau laser modulé multipoints, le masque de phase multipoints étant calculé pour répartir l'énergie du faisceau laser modulé multipoints en au moins deux points d'impact dans le plan focal de l'appareil de découpe,
- commander le déplacement du système de focalisation 50 pour faire coïncider le plan focal de l'appareil de découpe à la profondeur souhaitée pour le plan de découpe horizontal,
- piloter le scanner optique de balayage 40 pour déplacer les points d'impact de l'unique faisceau laser modulé multipoints le long d'un premier chemin de déplacement, et
- activer la source laser femtoseconde 10 (après stabilisation de la vitesse de déplacement du scanner).

### 2.5.2. Formation d'un plan de découpe vertical

Dans le cadre de la présente invention, la formation d'un plan de découpe vertical est réalisée comme suit.

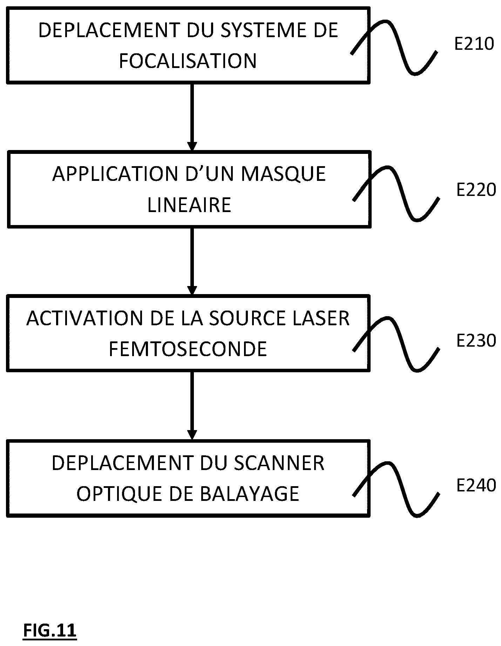

En référence à la figure 11, l'unité de commande 60 émet un signal de commande au système optique de focalisation 50 pour piloter son déplacement de sorte à positionner le plan focal de l'appareil de découpe à une profondeur donnée relativement à la position souhaitée pour le plan de découpe vertical (étape E210). En effet, comme indiqué précédemment, dans le cas d'un plan de découpe vertical, le plan de découpe peut être situé :
- au-dessus du plan focal de l'appareil de découpe si le masque de phase conique utilisé permet au SLM d'émuler un axicon positif (l'anneau de Bessel 33a est situé au-dessus de la ligne d'impact utilisée pour réaliser la découpe), dans ce cas l'unité de commande 60 pilote le système optique de focalisation 50 pour focaliser le faisceau laser modulé et dévié 410 à une profondeur désirée inférieure à la profondeur maximale du plan de découpe vertical à réaliser,
- au-dessous du plan focal de l'appareil de découpe dans le cas où le masque de phase conique utilisé permet au SLM d'émuler un axicon négatif (l'anneau de Bessel 33a est situé au-dessous de la ligne d'impact utilisée pour réaliser la découpe) dans ce cas l'unité de commande 60 pilote le système optique de focalisation 50 pour focaliser le faisceau laser modulé et dévié 410 à une profondeur désirée supérieure à la profondeur maximale du plan de découpe à réaliser.

L'unité de commande 60 transmet un masque de phase conique (i.e. consigne de modulation axiconique) au système de mise en forme 30 pour produire un faisceau laser modulé de Bessel (étape E220).

L'unité de commande 60 active également le déplacement du scanner optique de balayage 40 jusqu'à une position initiale du deuxième chemin optique de balayage.

Lorsque :
- le système de focalisation 50 et le scanner optique 40 sont en position,
- le masque de phase conique est chargé dans le système de mise en forme 30, et que
- la vitesse de pivotement du (ou des) miroir(s) du scanner optique 40 est constante, l'unité de commande 60 active la source laser femtoseconde 10 pour émettre une impulsion laser (étape E230).

La source laser femtoseconde 10 génère un faisceau laser 110 qui traverse le système de mise en forme 30. Le système de mise en forme 30 module la phase du faisceau laser pour produire un unique faisceau laser modulé de type Bessel.

Le faisceau laser modulé de type Bessel 310 sort du système de mise en forme 30 et entre dans le scanner optique 40 qui dévie le faisceau laser modulé de type Bessel 310.

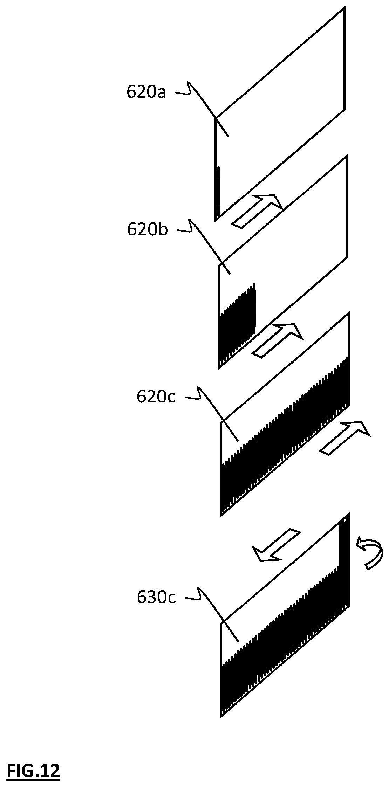

Le faisceau laser modulé et dévié 410 entre dans le système optique de focalisation 50 qui focalise le faisceau. Au-dessus ou au-dessous du plan focal, la consigne de modulation (i.e. le masque de phase conique) appliquée au système de mise en forme 30 permet de répartir l'énergie en une ligne d'impact. Cette ligne d'impact produit une bulle de gaz oblongue s'étendant parallèlement à l'axe optique A-A' de l'appareil de découpe, comme illustré à l'étape 620a de la figure 12.

Le laser femtoseconde 1 continue d'émettre d'autres impulsions sous forme de faisceau laser à une cadence déterminée. Entre chaque impulsion le (ou les) miroir(s) pivote(nt) d'un certain angle, ce qui a pour conséquence de déplacer la ligne d'impact et de former une nouvelle bulle de gaz oblongue le long du deuxième chemin optique (étape E240). Cette nouvelle bulle de gaz est adjacente à la bulle de gaz oblongue précédemment formée, comme illustré à l'étape 620b de la figure 12 après un certain nombre d'impulsions, formant ainsi un segment de plan de découpe vertical.

Les opérations de pilotage de la source laser femtoseconde 10 et du scanner optique de balayage 40 sont répétées pour former le plan de découpe vertical.

Plus précisément, le pivotement du (ou des) miroirs entre chaque impulsion de la source laser femtoseconde 10 a pour conséquence de déplacer la ligne d'impact et de produire de nouvelles bulles de gaz oblongues décalées par rapport aux précédentes, jusqu'à former un pan de découpe dans le plan de découpe, comme illustré à l'étape 620c de la figure 12. Une fois qu'un pan de découpe a été complété, l'unité de commande 60 désactive la source laser femtoseconde 10, et commande le déplacement du système optique de focalisation pour la réalisation d'un deuxième pan de bulles de gaz oblongues au-dessus du premier pan, puis commande à nouveau le redémarrage du pivotement du (ou des) miroir(s) dans le sens inverse et active à nouveau la source laser femtoseconde 10, comme illustré à l'étape 630c de la figure 12.

Le faisceau laser 110 traverse le système de mise en forme 30, le scanner optique 40 et le système optique de focalisation 50. Un nouveau pan de bulles de gaz oblongues - situé au-dessus du pan précédent et s'étendant dans le même plan que le pan précédent - se forme dans le plan de découpe.

Lorsque le scanner optique 40 a balayé toutes les positions du deuxième chemin de déplacement, le plan de découpe vertical est terminé.

En résumé, pour la réalisation d'un plan de découpe vertical, l'unité de commande 60 est configurée pour :
- appliquer au système de mise en forme 30, une consigne de modulation axiconique afin de produire un faisceau laser modulé de type Bessel à partir du faisceau laser gaussien, ladite consigne de modulation comportant un masque de phase 314, 315 émulant un axicon appliqué sur le modulateur spatial de lumière (SLM), ledit faisceau laser modulé de type Bessel créant une ligne d'impact permettant de générer une bulle de gaz oblongue dans le tissu,
- piloter le scanner optique de balayage 40 pour déplacer la ligne d'impact du faisceau laser modulé de type Bessel le long d'un deuxième chemin optique de déplacement pour former un plan vertical constitué d'un ensemble de bulles de gaz oblongues adjacentes.

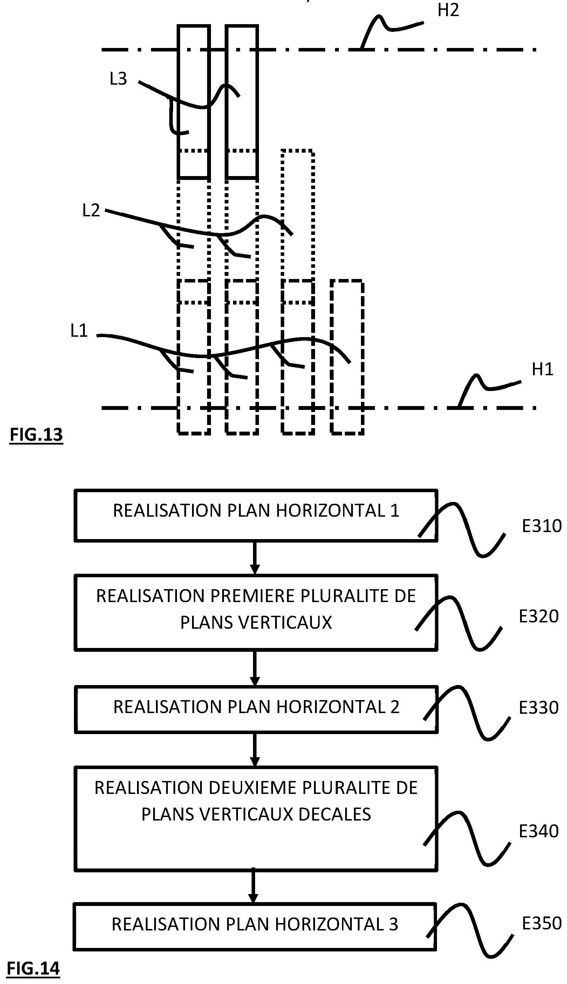

En référence à la figure 13, on a illustré partiellement un ensemble de lignes d'impact L1, L2, L3 utilisées pour réaliser des pans de bulles de gaz oblongues empilés et s'étendant dans un même plan de découpe verticale situé entre deux plans de découpe horizontaux H1, H2. Comme le montre la figure 13, les lignes d'impact utilisées pour former deux pans successifs empilés se superposent partiellement. En effet, les inventeurs ont découvert que les portions d'extrémités d'une ligne d'impact ne présentent pas une énergie suffisante pour former la bulle de gaz oblongue. C'est pourquoi les inventeurs proposent un recouvrement des lignes d'impact utilisées pour réaliser des pans de bulles de gaz oblongues empilés.

En particulier, après la réalisation d'un plan de découpe horizontal H1, les plans de découpe verticaux situés au-dessus du plan de découpe horizontal H1 sont réalisés en empilant des pans de bulles de gaz oblongues successifs, par exemple des premier, deuxième et troisième pans dans le mode de réalisation illustré à la figure 13.

Pour la réalisation du premier pan (i.e. le pan le plus proche du plan de découpe horizontal H1), l'unité de commande 60 est configurée pour piloter le système optique de focalisation 50 afin de positionner le plan focal de l'appareil de découpe à une distance prédéfinie non nulle du premier plan de découpe horizontal. Cette distance prédéfinie est inférieure à la longueur de la ligne d'impact L1 du faisceau laser modulé de type Bessel. Notamment, la distance prédéfinie peut être comprise entre $\frac{1}{5}$ et ⅓ de la longueur de la ligne d'impact. Ainsi, chaque ligne d'impact L1 utilisée pour la réalisation du premier pan intersecte partiellement le plan de découpe horizontal H1. L'unité de commande 60 pilote ensuite le scanner optique de balayage 40 pour déplacer la ligne d'impact L1 le long du deuxième chemin optique de déplacement pour former le premier pan de bulles de gaz oblongues adjacentes.

Pour la réalisation du deuxième pan (i.e. le pan situé au-dessus du premier pan), l'unité de commande 60 est configurée pour piloter le système optique de focalisation 50 afin de positionner le plan focal de l'appareil de découpe à la distance prédéfinie du premier pan de bulles de gaz oblongues. Ainsi, chaque ligne d'impact L2 utilisée pour la réalisation du deuxième pan recouvre partiellement les bulles de gaz oblongues du premier pan. L'unité de commande 60 pilote ensuite le scanner optique de balayage 40 pour déplacer la ligne d'impact L2 le long du deuxième chemin optique de déplacement pour former le deuxième pan de bulles de gaz oblongues adjacentes.

Pour la réalisation du troisième pan (i.e. le pan le plus éloigné du plan de découpe horizontal H1), l'unité de commande 60 est configurée pour piloter le système optique de focalisation 50 afin de positionner le plan focal de l'appareil de découpe à la distance prédéfinie du deuxième pan de sorte qu'une portion d'extrémité de la ligne d'impact L3 soit en contact avec les bulles de gaz oblongues du deuxième pan.

Ceci permet de garantir que les ponts tissulaires entre les différents pans empilés soient suffisamment étroits pour assurer une dissection par le praticien d'une qualité acceptable sur toute la hauteur de chaque plan de découpe vertical.

### 2.5.3. Formation de plans de découpe horizontaux et verticaux empilés pour créer des cubes de tissu

On va maintenant décrire plus en détails le principe de fonctionnement de l'appareil de découpe en référence à la destruction d'un cristallin dans le cadre d'une opération de la cataracte.

Pour partitionner le cristallin en cubes susceptibles d'être aspirés par une canule d'aspiration, des plans de découpe horizontaux et verticaux sont formés en commençant par le plan de découpe horizontal le plus profond dans le cristallin et en empilant les plans de découpe verticaux et horizontaux successifs jusqu'au plan de découpe horizontal le plus superficiel dans le cristallin.

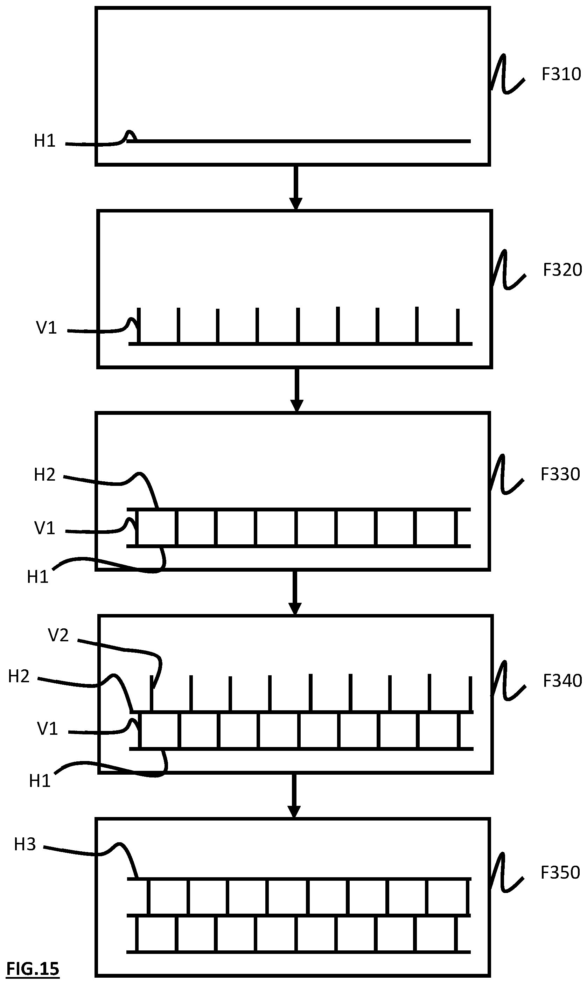

En référence aux figures 14 et 15, le plan de découpe horizontal H1 le plus profond est réalisé dans une première étape (E310, F310). L'unité de commande 60 :
- applique un masque de phase multipoints au système de mise en forme 30 pour produire un faisceau laser modulé multipoints,
- commande le déplacement du système de focalisation 50 pour faire coïncider le plan focal de l'appareil de découpe avec le plan de découpe le plus profond souhaité,
- pilote le déplacement du scanner optique de balayage jusqu'à une position cible le long du premier chemin optique (par exemple en créneau), et
- active la source laser femtoseconde 10 (après stabilisation de la vitesse de déplacement du scanner et l'atteinte de la position cible).

Une succession de tirs est réalisée dans le plan focal de l'appareil de découpe. A chaque tir, plusieurs points d'impact focalisent simultanément dans le plan focal. Chaque point d'impact forme une bulle de gaz. Le scanner optique permet de déplacer les plusieurs points d'impacts dans le plan focal entre chaque tir. Lorsque toute la surface du plan de découpe horizontal est recouverte de bulles de gaz, le plan de découpe horizontal H1 est terminé.

Dans une deuxième étape (E320 et F320), un premier ensemble de plans de découpe verticaux adjacents V1 est réalisé avec l'appareil de découpe. Pour chaque plan de découpe vertical V1, l'unité de commande 60 :
- applique un masque de phase conique au système de mise en forme 30 pour produire un faisceau laser modulé de Bessel,
- commande le déplacement du système de focalisation 50 pour positionner la ligne d'impact dans le plan de découpe (le plan de focalisation étant au-dessus ou en dessous du plan de découpe selon que l'axicon émulé sur le système de mise en forme soit un axicon positif ou négatif),
- pilote le déplacement du scanner optique de balayage jusqu'à une position cible le long du deuxième chemin optique (par exemple un segment), et
- active la source laser femtoseconde 10 (après stabilisation de la vitesse de déplacement du scanner et atteinte de la position cible).

Une succession de tirs est réalisée. A chaque tir, une ligne d'impact est générée. Chaque ligne d'impact forme une bulle de gaz oblongue selon l'axe optique de propagation du faisceau laser modulé. Le scanner optique de balayage 40 permet de déplacer la ligne d'impact au-dessus/en-dessous du plan focal entre chaque tir. Lorsque tout le deuxième chemin de déplacement est recouvert de bulles de gaz, le plan de découpe vertical V1 est terminé.

Si la profondeur de la ligne d'impact est inférieure à la profondeur souhaitée pour le plan de découpe vertical, alors l'unité de commande 60 peut contrôler le scanner optique de balayage 40 et le système optique de focalisation 50 pour déplacer la ligne d'impact le long du deuxième chemin optique en faisant varier la profondeur du plan focal de l'appareil de découpe entre l'aller et le retour.

On obtient ainsi plusieurs plans de découpe verticaux V1 au-dessus du plan de découpe horizontal H1 initial. Ces plans de découpe V1 sont répartis en 2 sous-groupes (un premier sous-groupe de plans 107' et un deuxième sous-groupe de plans 107" sur la figure 2) formant des plans parallèles au sein d'un même sous-groupe mais perpendiculaires pour 2 sous-groupes différents permettant ainsi d'obtenir des plans de découpe verticaux formant un quadrillage, chaque carré élémentaire représentant les parois latérales des cubes ainsi découpés.

Dans une troisième étape (E330, F330), un plan horizontal H2 intermédiaire est réalisé pour coiffer les plans de découpe verticaux V1. Ce plan de découpe horizontal H2 est réalisé selon la même méthode que celle décrite en référence à la première étape. On obtient ainsi des cubes de cristallin définis entre les plans horizontaux et verticaux réalisés aux première, deuxième et troisième étapes.

Dans une quatrième étape (E340, F340), un deuxième ensemble de plans de découpe verticaux adjacents V2 est réalisé avec l'appareil de découpe. Ce deuxième ensemble de plans de découpe verticaux V2 est décalé latéralement relativement au premier ensemble de plan de découpe verticaux V1. Pour ce faire, l'unité de commande 60 pilote le déplacement du scanner optique de balayage 40 le long d'un troisième chemin de déplacement différent - en particulier décalé latéralement - par rapport au deuxième chemin optique. Ainsi, aucun des plans de découpe verticaux V2 du deuxième ensemble n'est coplanaire avec un plan de découpe vertical V1 du premier ensemble.

Dans une cinquième étape (E350, F350), un plan horizontal H3 supérieur est réalisé selon la même méthode que celle décrite en référence à la première étape. On obtient ainsi un empilement de deux étages de cubes de cristallin, les cubes de cristallin du deuxième étage étant décalés latéralement par rapport aux cubes de cristallin du premier étage.

Les étapes précédentes peuvent être réitérées pour réaliser un empilement de plus de deux étages de cubes de cristallin, les cubes d'un étage étant décalé latéralement des cubes de cristallin situé à l'étage inférieur sur lequel ils reposent.

### 3. Conclusions

Le décalage latéral des plans de découpe verticaux situés au-dessus d'un plan de découpe horizontal relativement aux plans de découpe verticaux situés en dessous dudit plan de découpe horizontal permet de limiter la propagation des bulles de gaz jusqu'à la surface du tissu à traiter.

Comme indiqué précédemment, dans le cas d'une découpe de tissu oculaire, une telle propagation peut induire l'accumulation de gaz au-dessus du plan de découpe dans le cristallin, voir au-dessus du cristallin dans la chambre antérieure de l'œil et masquer le faisceau laser, empêchant ainsi la réalisation de la découpe dans la partie antérieure du cristallin.

Ainsi, l'invention permet de disposer d'un outil de découpe tridimensionnelle efficace permettant de réaliser des découpes en portions élémentaires de même taille et de faibles dimensions.

Le lecteur aura compris que de nombreuses modifications peuvent être apportées à l'invention décrite précédemment sans sortir matériellement des nouveaux enseignements et des avantages décrits ici.

## Revendications

1. Appareil de découpe d'un tissu humain ou animal, ledit appareil incluant une source laser femtoseconde (10) configurée pour émettre un faisceau laser gaussien sous forme d'impulsions et un dispositif de traitement du faisceau laser gaussien, le dispositif de traitement étant disposé en aval de la source laser femtoseconde (10), le dispositif de traitement comprenant :
- un système de mise en forme (30) positionné sur la trajectoire du faisceau laser gaussien, pour moduler la phase du front d'onde du faisceau laser gaussien, le système de mise en forme (30) comprenant un modulateur spatial de lumière (SLM) et étant configuré pour produire un faisceau laser modulé à partir du faisceau laser gaussien,
- un scanner optique de balayage (40) disposé en aval du système de mise en forme pour déplacer le faisceau laser modulé,
- un système optique de focalisation (50) en aval du système de mise en forme (30), pour focaliser le faisceau laser modulé dans un plan focal de l'appareil de découpe et pour déplacer le plan focal de l'appareil de découpe en une pluralité de positions le long d'un axe optique (A-A') de propagation du faisceau laser modulé, dans lequel le dispositif de traitement comprend en outre une unité de commande (60) pour piloter la source laser femtoseconde (10), le système de mise en forme (30), le scanner optique de balayage (40), et le système optique de focalisation (50) afin de réaliser des plans de découpe horizontaux et verticaux successifs, les plans de découpe horizontaux s'étendant perpendiculairement à l'axe optique (A-A') et les plans de découpe verticaux s'étendant parallèlement à l'axe optique (A-A'), ladite unité de commande (60) étant configurée pour :
- commander (E310) la réalisation d'un premier plan de découpe horizontal (H1),
- commander (E320) la réalisation d'une première pluralité de plans de découpe verticaux (V1) au-dessus du premier plan de découpe horizontal (H1),
- commander (E330) la réalisation d'un deuxième plan de découpe horizontal (H2) au-dessus de la première pluralité de plans de découpe verticaux (V1), le premier plan de découpe horizontal (H1) étant plus profond dans le tissu que le deuxième plan de découpe horizontal (H2),
- commander (E340) la réalisation d'une deuxième pluralité de plans verticaux (V2) au-dessus du deuxième plan horizontal (H2),
**caractérisé en ce que** la deuxième pluralité de plans de
découpe verticaux (V2) est décalée latéralement relativement à la première pluralité de plans verticaux (V1).

2. Appareil de découpe selon la revendication 1, ***dans lequel*** la deuxième pluralité de plans de découpe verticaux est décalée latéralement par rapport à la première pluralité de plans de découpe verticaux d'une distance comprise entre 5µm et 500 µm.

3. Appareil de découpe selon l'une quelconque des revendications 1 ou 2, ***dans lequel*** la deuxième pluralité de plans de découpe verticaux est décalée latéralement par rapport à la première pluralité de plans de découpe verticaux selon des premier et deuxième axes perpendiculaires à l'axe optique (A-A'), les premier et deuxième axes étant orthogonaux entre eux.

4. Appareil de découpe selon l'une quelconque des revendications 1 à 3, ***dans lequel*** pour la réalisation de chaque plan de découpe horizontal, l'unité de commande (60) est configurée pour :
o appliquer (E120) un masque de phase multipoints au système de mise en forme (30) pour produire un unique faisceau laser modulé multipoints, le masque de phase multipoints étant calculé pour répartir l'énergie du faisceau laser modulé multipoints en au moins deux points d'impact dans le plan focal de l'appareil de découpe,
o commander (E110) le déplacement du système de focalisation (50) pour faire coïncider le plan focal de l'appareil de découpe à la profondeur souhaitée pour le plan de découpe horizontal,
o piloter (E140) le scanner optique de balayage pour déplacer les points d'impact de l'unique faisceau laser modulé multipoints le long d'un premier chemin de déplacement,
o activer (E130) la source laser femtoseconde (10).

5. Appareil de découpe selon l'une quelconque des revendications 1 à 4, ***dans lequel*** pour la réalisation de chaque plan de découpe vertical (V1) de la première pluralité, l'unité de commande (60) est configurée pour :
o appliquer (E220) au système de mise en forme (30), une consigne de modulation axiconique afin de produire un faisceau laser modulé de type Bessel à partir du faisceau laser gaussien, ladite consigne de modulation comportant un masque de phase (314, 315) émulant un axicon appliqué sur le modulateur spatial de lumière (SLM), ledit faisceau laser modulé de type Bessel créant une ligne d'impact permettant de générer une bulle de gaz oblongue dans le tissu,
o piloter (E240) le scanner optique de balayage pour déplacer la ligne d'impact du faisceau laser modulé de type Bessel le long d'un deuxième chemin optique de déplacement pour former un ensemble de bulles de gaz oblongues adjacentes.

6. Appareil de découpe selon la revendication 5, ***dans lequel*** pour la réalisation de chaque plan de découpe vertical (V2) de la deuxième pluralité, l'unité de commande (60) est configurée pour :
o appliquer (E220) au système de mise en forme (30) la consigne de modulation axiconique,
o piloter (E240) le scanner optique de balayage pour déplacer la ligne d'impact du faisceau laser modulé de type Bessel le long d'un troisième chemin optique décalé latéralement relativement au deuxième chemin optique.

7. Appareil de découpe selon l'une des revendications 5 ou 6, ***dans lequel*** chaque plan de découpe vertical est composé d'un empilement de plusieurs ensembles de bulles de gaz oblongues adjacentes, l'unité de commande (60) étant configurée pour :
o piloter le système optique de focalisation (50) afin de positionner le plan focal de l'appareil de découpe à une distance prédéfinie non nulle du premier plan de découpe horizontal, ladite distance prédéfinie étant inférieure à la longueur de la ligne d'impact du faisceau laser modulé de type Bessel de sorte que la ligne d'impact intersecte partiellement le premier plan de découpe horizontal,
o piloter le scanner optique de balayage pour déplacer la ligne d'impact du faisceau laser modulé de type Bessel pour former un premier ensemble de bulles de gaz oblongues adjacentes,
o piloter le système optique de focalisation (50) afin de positionner le plan focal de l'appareil de découpe à la distance prédéfinie du premier ensemble de bulles de gaz oblongues adjacentes de sorte que la ligne d'impact intersecte partiellement le premier ensemble de bulles de gaz oblongues adjacentes,
o piloter le scanner optique de balayage pour déplacer la ligne d'impact du faisceau laser modulé de type Bessel pour former un deuxième ensemble de bulles de gaz oblongues adjacentes.

8. Appareil de découpe selon la revendication 7, dans lequel la distance prédéfinie est comprise en un $\frac{1}{5}$ et ⅓ de la longueur de la ligne d'impact.

## Patentansprüche

1. Vorrichtung zum Schneiden von menschlichem oder tierischem Gewebe, wobei die Vorrichtung eine Femtosekunden-Laserquelle (10), die dazu konfiguriert ist, einen gaußschen Laserstrahl in Impulsform auszusenden, und eine Vorrichtung zur Verarbeitung des gaußschen Laserstrahls umfasst, wobei die Verarbeitungsvorrichtung stromabwärts der Femtosekunden-Laserquelle (10) angeordnet ist, wobei die Verarbeitungsvorrichtung umfasst:
- ein Formgebungssystem (30), das im Strahlengang des gaußschen Laserstrahls positioniert ist, um die Phase der Wellenfront des gaußschen Laserstrahls zu modulieren, wobei das Formgebungssystem (30) einen räumlichen Lichtmodulator (SLM) umfasst und dazu konfiguriert ist, aus dem gaußschen Laserstrahl einen modulierten Laserstrahl zu erzeugen,
- einen optischen Abtastscanner (40), der stromabwärts des Formgebungssystems angeordnet ist, um den modulierten Laserstrahl zu verschieben,
- ein optisches Fokussiersystem (50) stromabwärts des Formgebungssystems (30), um den modulierten Laserstrahl in einer Fokalebene der Schneidvorrichtung zu fokussieren und um die Fokalebene der Schneidvorrichtung entlang einer optischen Ausbreitungsachse (A-A') des modulierten Laserstrahls in eine Vielzahl von Positionen zu verschieben,
wobei die Verarbeitungsvorrichtung ferner eine Steuereinheit (60) zum Ansteuern der Femtosekunden-Laserquelle (10), des Formgebungssystems (30), des optischer Abtastscanners (40) und des optischen Fokussiersystems (50) umfasst, um aufeinanderfolgende horizontale und vertikale Schneidebenen zu erzeugen, wobei sich die horizontalen Schneidebenen senkrecht zu der optischen Achse (A-A') erstrecken und sich die vertikalen Schneidebenen parallel zu der optischen Achse (A-A') erstrecken, wobei die Steuereinheit (60) dazu konfiguriert ist, um:
- die Realisierung einer ersten horizontalen Schneidebene (H1) zu steuern (E310),
- die Realisierung einer ersten Vielzahl vertikaler Schneidebenen (V1) oberhalb der ersten horizontalen Schneidebene (H1) zu steuern (E320),
- die Realisierung einer zweiten horizontalen Schneidebene (H2) oberhalb der ersten Vielzahl vertikaler Schneidebenen (V1) zu steuern (E330), wobei die erste Schneidebene (H1) tiefer als die zweite horizontale Schneidebene (H2) in dem Gewebe liegt,
- die Realisierung einer zweiten Vielzahl vertikaler Ebenen (V2) oberhalb der zweiten horizontalen Ebene (H2) zu steuern (E340),
**dadurch gekennzeichnet, dass** die zweite Vielzahl vertikaler Schneidebenen (V2) relativ zu der ersten Vielzahl vertikaler Ebenen (V1) seitlich versetzt ist.

2. Schneidvorrichtung nach Anspruch 1, wobei die zweite Vielzahl vertikaler Schneidebenen relativ zu der ersten Vielzahl vertikaler Schneidebenen in einem Abstand zwischen 5 µm und 500 µm seitlich versetzt ist.

3. Schneidvorrichtung nach einem der Ansprüche 1 oder 2, wobei die zweite Vielzahl vertikaler Schneidebenen relativ zu der ersten Vielzahl vertikaler Schneidebenen gemäß einer ersten und zweiten Achse, die senkrecht zu der optischen Achse (A-A') stehen, seitlich versetzt ist, wobei die erste und zweite Achse zueinander orthogonal sind.

4. Schneidvorrichtung nach einem der Ansprüche 1 bis 3, wobei zu der Realisierung jeder horizontalen Schneidebene die Steuereinheit (60) konfiguriert ist, um:
∘ eine Mehrpunkt-Phasenmaske auf das Formgebungssystem (30) anzuwenden (E120), um einen einzigen mehrpunktmodulierten Laserstrahl zu erzeugen, wobei die Mehrpunkt-Phasenmaske berechnet ist, um die Energie des mehrpunktmodulierten Laserstrahls auf mindestens zwei Auftreffpunkte in der Fokalebene der Schneidvorrichtung zu verteilen,
∘ die Bewegung des Fokussiersystems (50) so zu steuern (E110), dass die Fokalebene der Schneidvorrichtung in der für die horizontale Schneidebene gewünschten Tiefe zur Deckung gebracht wird,
∘ den optischen Abtastscanner so zu steuern (E140), dass die Auftreffpunkte des einzigen mehrpunktmodulierten Laserstrahls entlang einer ersten Bewegungsbahn verschoben werden,
∘ die Femtosekunden-Laserquelle (10) zu aktivieren (E130).

5. Schneidvorrichtung nach einem der Ansprüche 1 bis 4, wobei zur Realisierung jeder vertikalen Schneidebene (V1) der ersten Vielzahl die Steuereinheit (60) konfiguriert ist, um:
∘ auf das Formgebungssystem (30) einen axiconischen Modulationssollwert anzuwenden (E220), um aus dem gaußschen Laserstrahl einen modulierten Bessel-Laserstrahl zu erzeugen, wobei der Modulationssollwert eine Phasenmaske (314, 315) umfasst, die ein auf den räumlichen Lichtmodulator (SLM) angewendetes Axicon emuliert, wobei der modulierte Bessel-Laserstrahl eine Auftrefflinie erzeugt, die es ermöglicht, eine längliche Gasblase in dem Gewebe zu erzeugen,
∘ den optischen Abtastscanner zu steuern (E240), dass die Auftrefflinie des modulierten Bessel-Laserstrahls entlang eines zweiten optischen Bewegungswegs verschoben wird, um eine Gruppe benachbarter länglicher Gasblasen zu bilden.

6. Schneidvorrichtung nach Anspruch 5, wobei zur Realisierung jeder vertikalen Schneidebene (V2) der zweiten Vielzahl die Steuereinheit (60) konfiguriert ist, um:
∘ auf das Formgebungssystem (30) den axiconischen Modulationssollwert anzuwenden (E220),
∘ den optischen Abtastscanner so zu steuern (E240), dass die Auftrefflinie des modulierten Bessel-Laserstrahls entlang eines dritten optischen Weges verschoben wird, der relativ zu dem zweiten optischen Weg seitlich versetzt ist.

7. Schneidvorrichtung nach einem der Ansprüche 5 oder 6, wobei jede vertikale Schneidebenen aus einem Stapel mehrerer Gruppen benachbarter länglicher Gasblasen zusammengesetzt ist, wobei die Steuereinheit (60) konfiguriert ist, um:
∘ das optische Fokussiersystem (50) so zu steuern, dass die Fokalebene der Schneidvorrichtung in einem vordefinierten Abstand ungleich Null von der ersten horizontalen Schneidebene positioniert wird, wobei der vordefinierte Abstand kleiner als die Länge der Auftrefflinie des modulierten Bessel-Laserstrahls ist, so dass die Auftrefflinie die erste horizontale Schneidebene teilweise schneidet,
∘ den optischen Abtastscanner so zu steuern, dass die Auftrefflinie des modulierten Bessel-Laserstrahls verschoben wird, um eine erste Gruppe benachbarter länglicher Gasblasen zu bilden,
∘ das optische Fokussiersystem (50) so zu steuern, dass die Fokalebene der Schneidvorrichtung in dem vordefinierten Abstand zu der ersten Gruppe benachbarter länglicher Gasblasen positioniert wird, so dass die Auftrefflinie die erste Gruppe benachbarter länglicher Gasblasen teilweise schneidet,
∘ den optischen Abtastscanner so zu steuern, dass die Auftrefflinie des modulierten Bessel-Laserstrahls verschoben wird, um eine zweite Gruppe benachbarter länglicher Gasblasen zu bilden.

8. Schneidvorrichtung nach Anspruch 7, wobei der vordefinierte Abstand zwischen 1/5 und 1/3 der Länge der Auftrefflinie liegt.

## Claims

1. A cutting apparatus for a human or animal tissue, said apparatus including a femtosecond laser source (10) configured to emit a Gaussian laser beam in the form of pulses, and a processing device of the Gaussian laser beam, the processing device being arranged downstream of the femtosecond laser source (10), the processing device comprising:
- a shaping system (30) positioned on the trajectory of the Gaussian laser beam, to modulate the phase of the wavefront of the Gaussian laser beam, the shaping system (30) comprising a spatial light modulator (SLM) and being configured to produce a modulated laser beam from the Gaussian laser beam,
- a sweeping optical scanner (40) disposed downstream of the shaping system to move the modulated laser beam,
- an optical focusing system (50) downstream of the shaping system (30), for focusing the modulated laser beam in a focal plane of the cutting apparatus and for moving the focal plane of the cutting apparatus into a plurality of positions along an optical axis (A-A') of propagation of the modulated laser beam,
wherein the processing device further comprises a control unit (60) to drive the femtosecond laser source (10), the shaping system (30), the sweeping optical scanner (40) and the optical focusing system (50) in order to create successive horizontal and vertical cutting planes, the horizontal cutting planes extending perpendicularly to the optical axis (A-A') and the vertical cutting planes extending parallel to the optical axis (A-A'), said control unit (60) being configured to:
- control (E310) the creation of a first horizontal cutting plane (H1),
- control (E320) the creation of a first plurality of vertical cutting planes (V1) above the first horizontal cutting plane (H1),
- control (E330) the creation of a second horizontal cutting plane (H2) above the first plurality of vertical cutting planes (V1), the first horizontal cutting plane (H1) being deeper in the tissue than the second horizontal cutting plane (H2),
- control (E340) the creation of a second plurality of vertical planes (V2) above the second horizontal plane (H2),
**characterised in that** the second plurality of vertical cutting planes (V2) is laterally offset relative to the first plurality of vertical planes (V1).

2. The cutting apparatus according to claim 1, ***wherein*** the second plurality of vertical cutting planes is laterally offset relative to the first plurality of vertical cutting planes by a distance comprised between 5 µm and 500 µm.

3. The cutting apparatus according to any one of claims 1 or 2, ***wherein*** the second plurality of vertical cutting planes is laterally offset relative to the first plurality of vertical cutting planes along first and second axes perpendicular to the optical axis (A-A'), the first and second axes being orthogonal to each other.

4. The cutting apparatus according to any one of claims 1 to 3, ***wherein***, for the creation of each horizontal cutting plane, the control unit (60) is configured to:
∘ apply (E120) a multipoint phase mask to the shaping system (30) to produce a single multipoint modulated laser beam, the multipoint phase mask being calculated to distribute the energy of the multipoint modulated laser beam into at least two impact points in the focal plane of the cutting apparatus,
∘ control (E110) the movement of the focusing system (50) to make the focal plane of the cutting apparatus coincide with the desired depth for the horizontal cutting plane,
∘ drive (E140) the sweeping optical scanner to move the impact points of the single multipoint modulated laser beam along a first movement path,
∘ activate (E130) the femtosecond laser source (10).

5. The cutting apparatus according to any one of claims 1 to 4, ***wherein*** for the creation of each vertical cutting plane (V1) of the first plurality, the control unit (60) is configured to:
∘ apply (E220) an axicon modulation setpoint to the shaping system (30) in order to produce a Bessel-type modulated laser beam from the Gaussian laser beam, said modulation setpoint including a phase mask (314, 315) emulating an axicon applied to the spatial light modulator (SLM), said Bessel-type modulated laser beam creating a line of impact that allows generating an oblong gas bubble in the tissue,
∘ drive (E240) the sweeping optical scanner to move the line of impact of the Bessel-type modulated laser beam along a second optical movement path to form a set of adjacent oblong gas bubbles.

6. The cutting apparatus according to claim 5, ***wherein*** for the creation of each vertical cutting plane (V2) of the second plurality, the control unit (60) is configured to:
∘ apply (E220) the axicon modulation setpoint to the shaping system (30),
∘ drive (E240) the sweeping optical scanner to move the line of impact of the Bessel-type modulated laser beam along a third optical path laterally offset relative to the second optical path.

7. The cutting apparatus according to any of claims 5 or 6, ***wherein*** each vertical cutting plane is composed of a stack of several sets of adjacent oblong gas bubbles, the control unit (60) being configured to:
∘ drive the optical focusing system (50) in order to position the focal plane of the cutting apparatus at a predefined non-zero distance from the first horizontal cutting plane, said predefined distance being smaller than the length of the line of impact of the Bessel-type modulated laser beam such that the line of impact partially intersects the first horizontal cutting plane,
∘ drive the sweeping optical scanner to move the line of impact of the Bessel-type modulated laser beam to form a first set of adjacent oblong gas bubbles,
∘ drive the optical focusing system (50) in order to position the focal plane of the cutting apparatus at the predefined distance from the first set of adjacent oblong gas bubbles such that the line of impact partially intersects the first set of adjacent oblong gas bubbles,
∘ drive the sweeping optical scanner to move the line of impact of the Bessel-type modulated laser beam to form a second set of adjacent oblong gas bubbles.

8. The cutting apparatus according to claim 7, wherein the predefined distance is comprised between $\frac{1}{5}$ and ⅓ of the length of the line of impact.
